# EUROPEAN PATENT APPLICATION

(11) **EP 2 612 591 A1**
(43) Date of publication of application: **10.07.2013**
(21) Application number: 11821279.4
(22) Date of filing: 24.08.2011
(51) Int. Cl.: A61B 5/00, G06F 3/048, G06Q 50/00

(54) **MEDICAL INFORMATION DISPLAY DEVICE, METHOD AND PROGRAM**

(30) Priority: 30.08.2010 JP 2010191853
(71) Applicant: FUJIFILM Corporation, Tokyo 106-8620 (JP)
(72) Inventor: ASAMI, Masahiro, Tokyo 107-0052 (JP); SHIRASAKA, Hajime, Tokyo 107-0052 (JP)
(74) Representative: Klunker . Schmitt-Nilson . Hirsch
(86) International application number: PCT/JP2011/004710
(87) International publication number: WO 2012/029255

(57) **Abstract**

[Problems to be Solved]

Allowing a user to obtain desired medical information more easily through a more intuitive operation.

[Means for Solving the Problems]

The obtaining condition input interface (32) receives a gesture input while a subject appearance image (human body icon (45)) is displayed, the obtaining condition identification unit (35) identifies, based on a type of the gesture determined by the gesture type analysis unit (33) and a region of the subject corresponding to the gesture identified by the gesture region analysis unit (34), a medical information obtaining condition for obtaining medical information corresponding to the gesture, the medical information obtaining unit (36) selectively obtains medical information satisfying the identified medical information obtaining condition from the medical information database (53) storing a plurality of sets of medical information, and the medical information display control unit (36) displays the obtained medical information on the display means.

## Description

### Technical field

The present invention relates to a technology that allows a user to display desired medical information using an intuitive user interface, such as a touch panel.

### Background art

In medical sites, a wide variety of medical information is generated, including waveform information, such as electrocardiograms, electroencephalograms, and the like, numerical information, such as blood pressures, body temperatures, and the like, and textual information, such as various examination reports, medical records, and the like, as well as image information obtained by various modalities, such as CT, MRI, US, PET, and the like.

Some medical institutions have established a system for managing such medical information. For example, such medical information is stored in a database as electronic data, then medical information desired by a user is selected in response to a request from a client terminal, and the selected information is displayed on a display device connected to the client terminal.

In order to improve the operability of selection and display of such medical information, various user interfaces are proposed. For example, such a user interface is known as described, for example, in Japanese Unexamined Patent Publication No. 2003-260030 in which the user is allowed to specify a region within a human shape or within an image representing a portion of a body displayed on a display screen using a pointing device and, if specified, medical image information of a diseased region within the specified area or around there is extracted from a medical database and a list of the extracted medical image information is displayed.

Further, another user interface is known as described, for example, in Japanese Unexamined Patent Publication No. 2009-119000 in which the user is allowed to draw a reference line in an axial cross-sectional image by a touch operation using an input device having a touch screen display connected to and used with a medical image processing workstation and, when drawn, a coronal cross-sectional image with the reference line as the cutting plane is generated and displayed.

### Disclosure of the Invention

The user interface described in Japanese Unexamined Patent Publication No. 2003-260030, however, is an interface intended to obtain medical image information as much as possible by specifying one region and the use of the interface causes a list of very large amount of medical information to be displayed and it may become sometimes difficult to intuitively narrow down the range of required medical information appropriately and rapidly in view of the fact that a wide variety of medical information is generated in medical sites. The user interface described in Japanese Unexamined Patent Publication No. 2009-119000 is an interface intended to switch an already selected image to another and not for appropriately narrowing down the range of required medical information.

The present invention has been developed in view of the circumstances described above, and it is an object of the present invention to provide a medical information display apparatus, method, and program that allows a user to obtain desired medical information more easily through a more intuitive operation.

A medical information display apparatus of the present invention is an apparatus, including:
a display means for displaying given information;
a gesture input means for detecting a gesture operation performed on a display surface of the display means and outputting gesture information representing a content of the detected gesture operation;
a first display control means for displaying a subject appearance image representing an appearance of a subject at a predetermined display position of the display means based on image data of the subject appearance image, wherein each position of the image is related to region identification information for identifying a region of the subject;
a gesture type analysis means for determining, based on gesture information outputted according to a gesture operation detected by the gesture input means while the subject appearance image is displayed, a gesture type representing to which of a plurality of predetermined gesture operation patterns the detected gesture operation correspond;
a gesture region analysis means for identifying a gesture region which is a region of the subject corresponding to the detected gesture operation based on information of the display position of the subject appearance image, the region identification information related to the subject appearance image data, and gesture position information representing a position on which the gesture operation has been performed included in the gesture information outputted according to the gesture operationwhile the subject appearance image is displayed;
an obtaining condition identification means for identifying a medical information obtaining condition for obtaining medical information of the subject corresponding to the gesture operation performed while the subject appearance image is displayed based on the gesture type and the gesture region;
a medical information obtaining means for selectively obtaining medical information satisfying the identified medical information obtaining condition from a medical information storage means storing a plurality of sets of medical information; and
a second display control means for displaying the obtained medical information on the display means.

A medical information display system of the present invention is a system in which a medical information supply apparatus for selectively supplying medical information of a subject based on a given medical information obtaining condition and the medical information display apparatus of the present invention are communicatively linked via a network.

Here, the medical information supply apparatus may include: a medical information storage means storing a plurality of sets of medical information in a data structure that allows selection of medical information based on a given medical information obtaining condition; an obtaining condition receiving means for receiving a medical information obtaining condition from the medical information display apparatus; a medical information retrieval means for obtaining medical information satisfying the received medical information obtaining condition from the medical information storage means; and a medical information transmission means for transmitting the obtained medical information to the medical information display apparatus that has transmitted the medical information obtaining condition.

A medical information display method of the present invention is a method, including:
a step of displaying a subject appearance image representing an appearance of a subject at a predetermined display position of a display means based on image data of the subject appearance image, wherein each position of the image is related to region identification information for identifying a region of the subject;
a step of detecting a gesture operation performed on a display surface of the display means while the subject appearance image is displayed and outputting gesture information representing a content of the detected gesture operation;
a step of determining a gesture type representing to which of a plurality of predetermined gesture operation patterns the detected gesture operation correspond based on the outputted gesture information;
a step of identifying a gesture region which is a region of the subject corresponding to the detected gesture operation based on information of the display position of the subject appearance image, the region identification information related to the subject appearance image data, and gesture position information representing a position on which the gesture operation has been performed included in the gesture information outputted according to the gesture operation;
a step of identifying a medical information obtaining condition for obtaining medical information of the subject corresponding to the gesture operation performed while the subject appearance image is displayed based on the gesture type and gesture region;
a step of selectively obtaining medical information satisfying the identified medical information obtaining condition from a medical information storage means storing a plurality of sets of medical information; and
a step of displaying the obtained medical information on the display means.

A medical information display control program of the present invention is a program for causing a computer to perform:
a step of displaying a subject appearance image representing an appearance of a subject at a predetermined display position of a display means based on image data of the subject appearance image, wherein each position of the image is related to region identification information for identifying a region of the subject;
a step of detecting a gesture operation performed on a display surface of the display means while the subject appearance image is displayed and outputting gesture information representing a content of the detected gesture operation;
a step of determining a gesture type representing to which of a plurality of predetermined gesture operation patterns the detected gesture operation correspond based on the outputted gesture information;
a step of identifying a gesture region which is a region of the subject corresponding to the detected gesture operation based on information of the display position of the subject appearance image, the region identification information related to the subject appearance image data, and gesture position information representing a position on which the gesture operation has been performed included in the gesture information outputted according to the gesture operation;
a step of identifying a medical information obtaining condition for obtaining medical information of the subject corresponding to the gesture operation performed while the subject appearance image is displayed based on the gesture type and gesture region;
a step of selectively obtaining medical information satisfying the identified medical information obtaining condition from a medical information storage means storing a plurality of sets of medical information; and
a step of displaying the obtained medical information on the display means.

In the present invention, a touch panel type input means may be used to input a gesture.

The subject appearance image may be an image schematically representing the subject.

The subject appearance image may be displayed by changing the appearance of the subject in the subject appearance image to a predetermined appearance according to the detected gesture operation based on the gesture type and/or the gesture region corresponding to the detected gesture operation while the subject appearance image is displayed.

Otherwise, the subject appearance image may be displayed by changing, based on a first gesture type determined with respect to a first gesture operation detected while the subject appearance image is displayed and/or a first gesture region identified with respect to the first gesture operation, the appearance of the subject in the subject appearance image to a predetermined appearance according to the detected gesture operation and, based on at least some of the first gesture type, the first gesture region, a second gesture type determined by the gesture type determination means with respect to a second gesture operation detected while the changed subject appearance image is displayed, and a second gesture region identified by the gesture region identification means with respect to the second gesture operation, a medical information obtaining condition corresponding to the first and second gesture operations may be identified.

When identifying a medical information obtaining condition, the medical information obtaining condition may be identified based on reference data in which medical information obtaining condition is related to a combination of gesture type and gesture region.

The reference data may be data in which one or more medical information obtaining conditions are related to a pair of gesture type and gesture region and if two or more medical information obtaining conditions are related to the pair, a priority may further be related to each of the plurality of medical information obtaining conditions.

Further, an arrangement may be adopted in which the reference data are allowed to be edited.

Medical information satisfying a given condition may be pre-obtained from the medical information storage means. Further, each region of the subject represented in the subject appearance image may be displayed such that a region whose medical information is included in the pre-obtained medical information differs in appearance from a region whose medical information is not included in the pre-obtained medical information.

When displaying medical information, if a plurality of sets of medical information with respect to examinations of the same type with different examination times is obtained by the medical information obtaining means, the plurality of sets of medical information may be displayed in a comparable manner.

When a medical image representing the subject is obtained from the medical information storage means, predetermined image processing may be performed on the obtained medical image to obtain a medical image satisfying the medical information obtaining condition, as required.

When a plurality of sets of medical information satisfying the medical information obtaining condition is obtained, the plurality of sets of medical information may be list-displayed to receive selection of medical information to be displayed, and the selected medical information may be displayed.

Here, when list-displaying the plurality of extracted sets of medical information, the plurality of sets of medical information may be displayed in the form of thumbnails or icons.

According to the present invention, the following are performed: receiving, while a subject appearance image is displayed, a gesture operation performed on the display surface of the image; determining a gesture type representing to which of a plurality of predetermined gesture operation patterns the detected gesture operation correspond based on gesture information representing a content of the gesture operation; identifying a gesture region which is a region of the subject corresponding to the detected gesture operation based on information of the display position of the subject appearance image, region identification information related to the subject appearance image data, and gesture position information representing a position on which the gesture operation has been performed, identifying a medical information obtaining condition for obtaining medical information of the subject corresponding to the gesture operation performed while the subject appearance image is displayed based on the gesture type and gesture region; selectively obtaining medical information satisfying the identified medical information obtaining condition from a medical information storage means storing a plurality of sets of medical information; and displaying the obtained medical information on the display means. Accordingly, this allows the user to obtain desired medical information more easily through a more intuitive operation.

### Brief Description of Drawings

Figure 1 illustrates a configuration of a medical information integration system, which includes a medical information display apparatus according to an embodiment of the present invention, and peripheral systems.
Figure 2 schematically illustrates an external view of the medical information display apparatus according to an embodiment of the present invention.
Figure 3 is a block diagram of the medical information display apparatus according to an embodiment of the present invention, illustrating major components thereof.
Figure 4 is a block diagram of the medical information display apparatus and medical information management server, schematically illustrating the functions implemented therein in first and second embodiments.
Figure 5 is a flowchart illustrating a processing flow for displaying medical information performed in the medical information integration system in the first and second embodiments of the present invention.
Figure 6 illustrates, by way of example, a data structure and an example of registered data in the first embodiment of the present invention.
Figure 7 illustrates, by way of example, a human body icon.
Figure 8 illustrates, by way of example, a relationship between position information of each position of the human body icon and region information.
Figure 9A schematically illustrates, by way of example, a knife gesture performed on the human body icon.
Figure 9B schematically illustrates that the shape of the human body icon is changed by the knife gesture.
Figure 10A schematically illustrates, by way of example, a specification gesture performed on the human body icon.
Figure 10B schematically illustrates that the region specified by the specification gesture is highlighted.
Figure 11A schematically illustrates, by way of example, a seize gesture performed on the human body icon.
Figure 11B schematically illustrates that the region specified by the seize gesture is seized out.
Figure 12A schematically illustrates, by way of example, a hammer gesture performed on the human body icon.
Figure 12B schematically illustrates that the shape of the human body icon is changed by the hammer gesture.
Figure 13A illustrates, by way of example, an obtaining condition table setting.
Figure 13B illustrates, by way of example, an interface for editing the obtaining condition table.
Figure 14 schematically illustrates, by way of example, a CT cross-sectional image.
Figure 15 schematically illustrates, by way of example, electrocardiographic waveform data.
Figure 16 schematically illustrates, by way of example, a volume rendering image representing a heart.
Figure 17 schematically illustrates, by way of example, a combined display of volume rendering images of a skull, a brain parenchyma, and brain blood vessels.
Figure 18 illustrates, by way of example, an obtaining condition input user interface in the second embodiment of the present invention.
Figure 19 schematically illustrates, by way of example, an obtaining condition that combines a knife gesture with an upward specification gesture.
Figure 20 illustrates, by way of example, an obtaining condition table setting corresponding to the input example of the obtaining condition that combines a knife gesture with an upward specification gesture.
Figure 21 illustrates, by way of example, medical information registered in the medical information database corresponding to the input example of the obtaining condition combining a knife gesture with an upward specification gesture.
Figure 22 schematically illustrates, by way of example, a knife gesture performed on the human body icon in an up-to-down direction.
Figure 23A schematically illustrates, by way of example, a rotation gesture in a left-right direction.
Figure 23B schematically illustrates, by way of example, a knife gesture in an up-to-down direction to a laterally oriented human body icon.
Figure 24 illustrates, by way of example, an obtaining condition table setting corresponding to the obtaining condition combining the knife gesture in an up-to-down direction with the rotation gesture in a left-right direction.
Figure 25 illustrates, by way of example, medical information registered in the medical information database corresponding to the input example of the obtaining condition combining the knife gesture in an up-to-down direction with the rotation gesture in a left-right direction.
Figure 26 schematically illustrates, by way of example, an input of an obtaining condition combining a seize gesture with a specification gesture.
Figure 27 illustrates, by way of example, an obtaining condition table setting corresponding to the input example of the obtaining condition combining the seize gesture with the specification gesture.
Figure 28 is a block diagram of the medical information display apparatus and medical information management server, schematically illustrating the functions implemented therein in a third embodiment of the present invention.
Figure 29 is a flowchart illustrating a processing flow for displaying medical information performed in the medical information integration system in the third embodiment of the present invention.
Figure 30 is a block diagram of the medical information display apparatus and medical information management server, schematically illustrating the functions implemented therein in a fourth embodiment of the present invention.
Figure 31 is a flowchart illustrating a processing flow for displaying medical information performed in the medical information integration system in the fourth embodiment of the present invention.

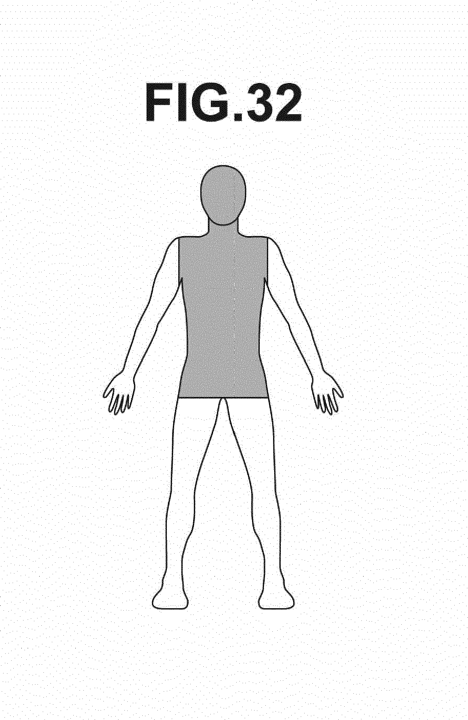

Figure 32 illustrates, by way of example, a display of human body icon in the fourth embodiment.
Figure 33 illustrates, byway of example, medical information registered in the medical information database corresponding to a fifth embodiment of the present invention.
Figure 34 illustrates, by way of example, a comparative display of medical information in the fifth embodiment of the present invention.
Figure 35 is a block diagram of the medical information display apparatus and medical information management server, schematically illustrating the functions implemented therein in a sixth embodiment of the present invention.
Figure 36 is a flowchart illustrating a processing flow for displaying medical information performed in the medical information integration system in the sixth embodiment of the present invention.
Figure 37A illustrates, by way of example, a medical information selection screen.
Figure 37B illustrates, by way of example, an alternative medical information selection screen.
Figure 38 is a block diagram of the medical information display apparatus and medical information management server, schematically illustrating the functions implemented therein in a seventh embodiment of the present invention.
Figure 39 is a flowchart illustrating a processing flow for displaying medical information performed in the medical information integration system in the seventh embodiment of the present invention.
Figure 40 illustrates, by way of example, medical information registered in the medical information database corresponding to the seventh embodiment of the present invention.
Figure 41 illustrates, by way of example, a structure and a setting of obtaining condition table in an eighth embodiment of the present invention.
Figure 42 schematically illustrates, by way of example, a display condition (layout) set in the obtaining condition table.
Figure 43 schematically illustrates, by way of example, a display of medical information in the eighth embodiment of the present invention.

### Best Mode for Carrying Out the Invention

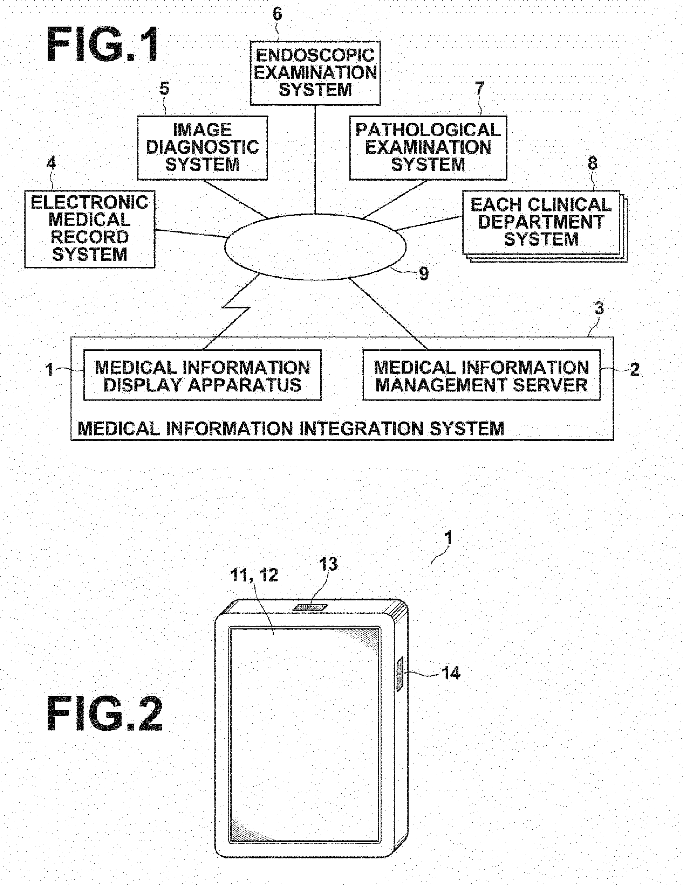

Figure 1 illustrates a configuration of a medical information integration system 3, which includes a medical information display apparatus 1 according to an embodiment of the present invention, and peripheral systems. As illustrated in the drawing, the medical information integration system 3 is communicatively linked to an electronic medical record system 4, an image diagnostic system 5, an endoscopic examination system 6, a pathological examination system 7, and an each clinical department system 8 via a network 9. The medical information integration system 3 includes the medical information display apparatus 1 according to an embodiment of the present invention and a medical information management server 2.

In the present embodiment, medical information generated in the electronic medical record system 4, the image diagnostic system 5, the endoscopic examination system 6, the pathological examination system 7, and the each clinical department system 8 is integrally collected and managed by the medical information management server 2. The medical information display apparatus 1 makes a request for desired medical information to the medical information management server 2 and displays medical information satisfying the request supplied from the medical information management server 2.

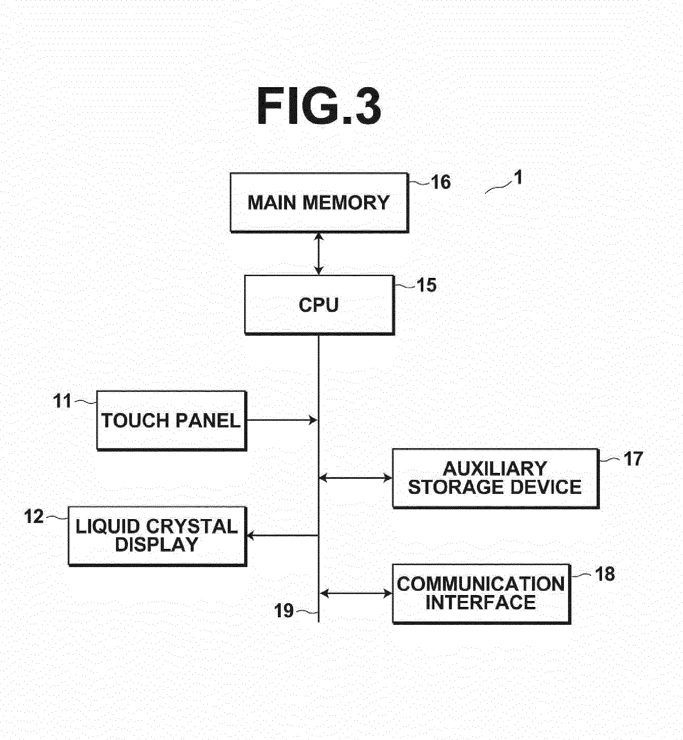

Figure 2 schematically illustrates an appearance of the medical information display apparatus 1 according to an embodiment of the present invention, and Figure 3 is a block diagram of the medical information display apparatus 1, illustrating major components thereof.

As illustrated in Figure 2, the medical information display apparatus 1 includes a liquid crystal display 12 integrally formed with a touch panel 11 on the front side with an antenna unit 13 for wireless communication and a power switch 14 on side surfaces.

As illustrated in Figure 3, the medical information display apparatus 1 is of a configuration in which a CPU 15, a main memory 16, an auxiliary storage device 17, the touch panel 11, the liquid crystal display 12, a communication interface 18, and the like are linked via a data bus.

The CPU 15 performs each processing by loading middleware, such as an operating system and the like, and each program, such as application software for obtaining and displaying medical information of the present invention, stored in the auxiliary storage device 17 to the main memory 16. This allows receiving of user input via the touch panel 11, input/output control, such as display control of various types of information, including medical information, on the liquid crystal display 12, communication via the communication interface 18, and the like.

As for the auxiliary storage device 17, a well-known flash memory drive (SSD: Solid State Drive) or a hard disk drive (HDD) is used. The auxiliary storage device 17 includes each program described above installed therein. The application software for displaying medical information of the present invention may be installed from a recording medium, such as CD-ROM or the like, using a disk drive connected to the medical information display apparatus 1 or installed after downloaded from a storage device of a server linked to the apparatus 1 via a network, such as the Internet or the like. Further, the auxiliary storage device 17 is used for temporarily storing medical information obtained from the medical information management server 2.

As for the touch panel 11, any known type may be used, including resistive type, capacitive type, electromagnetic type, surface elastic (ultrasonic) wave type, infrared type, and the like. In the present embodiment, a projected capacitive touch panel capable of detecting a multi-touch, i.e., touches at a plurality of positions, is used in order to explain a wide variety of gesture patterns. Touch operations on the touch panel 11 are performed with a finger of the user or with a predetermined pen or the like. The touch panel 11 detects the start of touching thereon, movement of the touched position, and end of the touching at a time interval defined by the control program, and outputs information of detected touch type and touched position at the time in a coordinate system of the touch panel 11. The term "start of touching" as used herein refers to a touching operation to a new position on the touch panel 11, the term "movement of touched position" refers to a moving operation of the touched position with the touch panel 11 being kept touched, and the term "end of touching" refers to a removing operation from the touch panel. This allows various gesture operations performed on the touch panel 11 to be detected. That is, a series of operations from the start of touching, movement of the touched position, and end of the touching is detected as one gesture operation, and touch type and position information detected at each time point of the series of operations is obtained as gesture information. The correspondence relationship between the coordinate system of the touch panel 11 and the coordinate system of the liquid crystal display 12 is identified through calibration at the time when the medical information display apparatus 1 is manufactured, so that a mutual coordinate conversion is possible. Hereinafter, the coordinate system of the liquid crystal display 12 and the coordinate system of the touch panel 11 are assumed to be the same coordinate system and referred to as the coordinate system of the display apparatus in order to simplify the description.

The communication interface 18 controls communication through a well-known mobile communication network, wireless LAN, and the like. In the present embodiment, communication with the medical information management server 2 is performed via the communication interface 18.

In the mean time, the medical information management server 2 is a computer having a medical information database. As for the hardware configuration, it includes an external storage device in addition to well-known hardware devices, including CPU, main memory, auxiliary storage device, I/O interface, communication interface, data bus, and the like. The medical information management server 2 is provided with application software for medical information registration in and extraction from the database, as well as a well-known operating system and database management software. Such software is installed from a recording medium, such as CD-ROM or the like, or after downloaded from a storage device of a server linked thereto via a network, such as the Internet or the like.

The electronic medical record system 4 employs a known computer system and is of a configuration in which, for example, a terminal of the each clinical department and an electronic medical record management server having an electronic medical record database in which electronic medical record information is stored are communicatively linked via a network. Electronic medical record information inputted from a terminal of each clinical department and the like is managed using the electronic medical record database. For example, the electronic medical record includes: patient information, such as name, date of birth, gender, and the like of a patient; examination history information, such as date of each examination received, contents, results, and the like; diagnostic history, such as date of diagnosis received, major complaint, determined disease name, and the like; and treatment history information, such as date of operation, procedure, or medication and the like. In the present embodiment, the electronic medical record database has a database structure in which a patient ID for identifying each patient is related to the aforementioned electronic medical record.

The image diagnostic system 5 also employs a known computer system and is of a configuration in which, for example, an image diagnostic medical workstation, an image management server having a database storing image data captured by modalities, such as CT, MRI, and the like, and an image interpretation report server having an image interpretation report database storing image interpretation reports of image interpretation results of the captured images are communicatively linked via a network. Here, the image diagnosis medical workstation is capable of performing known image processing such as MIP, MPR, CPR, volume rendering (VR), or the like according to the purpose or target of the diagnosis in combination with a known image analysis, such as bone extraction/elimination, blood vessel extraction, organ extraction, detection of abnormal tissue pattern, or the like, and these processed/analyzed images are also stored in the image database. The image data may include both two-dimensional images (pixel data) and three-dimensional images (voxel data), and both still images and moving images. In addition to the patient ID, the image database includes other auxiliary information related to each image, such as an image ID for identifying each image, modality information by which the image is obtained, region information of a subject in the image, and the like. The modality information is provided by the modality at the time of image generation. The region information of a subject may be provided by the modality at the time of image generation based on the examination order or the like or, if the image is a tomographic image, such as a CT image or the like, the region information may be provided by the image diagnosis medical workstation for each slice using a well-known region recognition technique, such as that described in Japanese Unexamined Patent Publication NO. 2008-259682. The image interpretation report database has a database structure in which each image interpretation report, patient ID, and image ID of an interpretation target image are related to each other. Each image data or image interpretation report may be indirectly related to the patient ID by way of examination ID for identifying each examination (imaging).

The endoscopic examination system 6 also employs a known computer system and includes an endoscopic examination management server with an endoscopic examination database having therein real endoscopic images obtained by various types of endoscopes, endoscopic examination reports which include summaries of endoscopic examination results, and the like related to the examination IDs and patient IDs, and access control to the endoscopic examination database is performed by the server.

The pathological examination system 7 also employs a known computer system and includes a pathological examination management server with a pathological examination database having therein microscope images obtained by pathological examinations, pathological examination reports which include summaries of pathological examination results, and the like related to examination IDs and patient IDs, and access control to the pathological examination database is performed by the server.

The each clinical department system 8 includes a management server of each clinical department with a database of each clinical department having therein examination data, examination reports, and the like unique to each clinical department related to the examination IDs and patient IDs, and access control to the database of each clinical department is performed by each server. The examination data unique to each clinical department may be, for example, electrocardiogram data and the like (waveforms, numerical values, or the like) if the clinical department is a cardiovascular department, auditory test data and the like (waveforms, numerical values, or the like) if the department is an otolaryngology department, or visual acuity test data, fundus examination data or the like (numerical values, or the like) if the department is an ophthalmology department.

In the present embodiment, the endoscopic examination system 6 and pathological examination system 7 are systems separate from the each clinical department system 8, but they may be integrated as a part of the each clinical department system 8. In this case, information of endoscopic examinations and pathological examinations is managed as examination data of each clinical department according to the content of each examination.

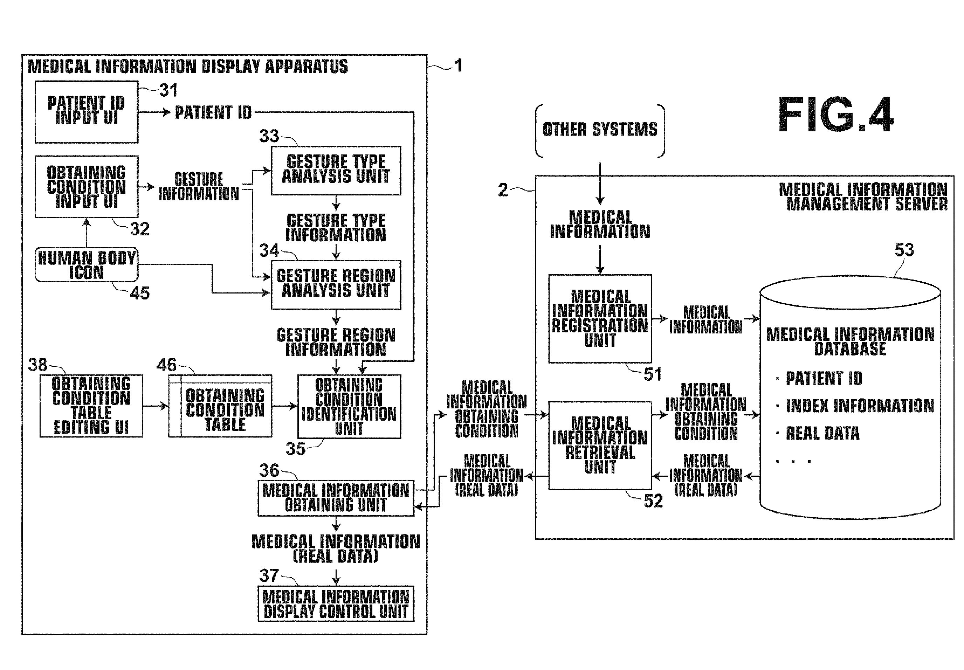

A first embodiment of the present invention is an embodiment in which medical information is obtained from the medical information management server 2 according to each of various types of touch panel operations performed in the medical information display apparatus 1 and displayed on the liquid crystal display 12. Figure 4 is a block diagram of the medical information display apparatus 1 and medical information management server 2, schematically illustrating the functions implemented therein in the first embodiment. As illustrated in the drawing, medical information display apparatus 1 of the present invention includes a patient ID input user interface (UI) 31, an obtaining condition input UI 32, a gesture type analysis unit 33, a gesture region analysis unit 34, an obtaining condition identification unit 35, medical information obtaining unit 36, medical information display control unit 37, a human body icon 45, and an obtaining condition table 46. The patient ID, gesture information, gesture region information, gesture type information, medical image obtaining condition, medical information (actual data) shown in the medical information display apparatus 1 are data written into or read from a predetermined area of the main memory 16 or auxiliary storage device 17 of the medical information display apparatus 1 by each of the processing units described above. In the mean time, the medical information management server 2 includes a medical information registration unit 51, a medical information retrieval unit 52, and a medical information database 53. The medical information registration condition, medical information, medical information obtaining condition, medical information (real data) shown in the medical information management server 2 are data written into or read from a predetermined area of the main memory or auxiliary storage device of the medical information management server 2, or an external storage device.

The medical information database 53 has a database structure in which patient ID, index information (to be described later) corresponding to medical information obtaining condition, and real data of the medical information are related.

The medical information registration unit 51 of the medical information management server 2 obtains medical information generated in other systems (the electric medical record system 4, the image diagnostic system 5, the endoscopic examination system 6, the pathological examination system 7, and the each clinical department system 8) at a predetermined time interval, extracts patient ID and index information from the obtained medical information, converts the obtained medical information to the data structure of the medical information database 53, and registers the information in the medical information database 53. This causes display target medical information for the medical information display apparatus 1 to be accumulated in the medical information database 53.

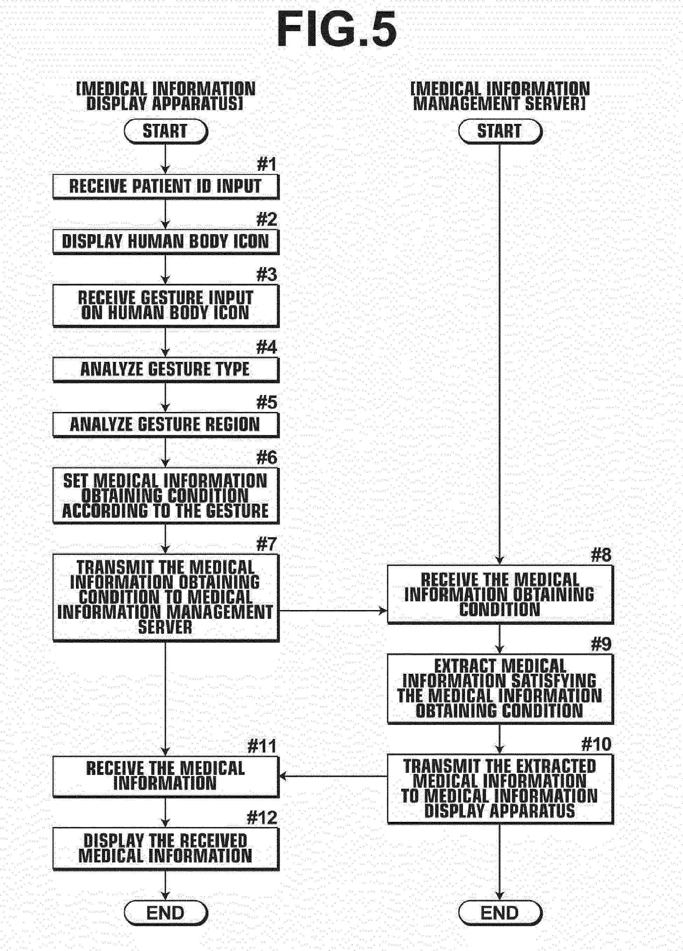

Figure 5 is a flowchart illustrating a processing flow for displaying medical information performed in the medical information integration system 3 in the first embodiment. Steps from #1 to #7 and steps from #11 to #12 are controlled by the main program of the application software executed in the medical information display apparatus 1. Hereinafter, an overall flow of the medical information display processing and individual steps performed by each processing unit of the medical information display apparatus 1 and medical information retrieval unit 52 of the medical information management server 2 will be described with reference mainly to Figures 4 and 5.

First, in the medical information display apparatus 1, the patient ID input UI 31 receives a patient ID and stores the inputted patient ID to a predetermined area of the main memory 16 (#1). More specifically, the patient ID is received, for example, using a software keyboard system in which an image of a keyboard or a numeric keypad is displayed on the liquid crystal display 12 and a key input displayed at the touched position on the touch panel 11 is received.

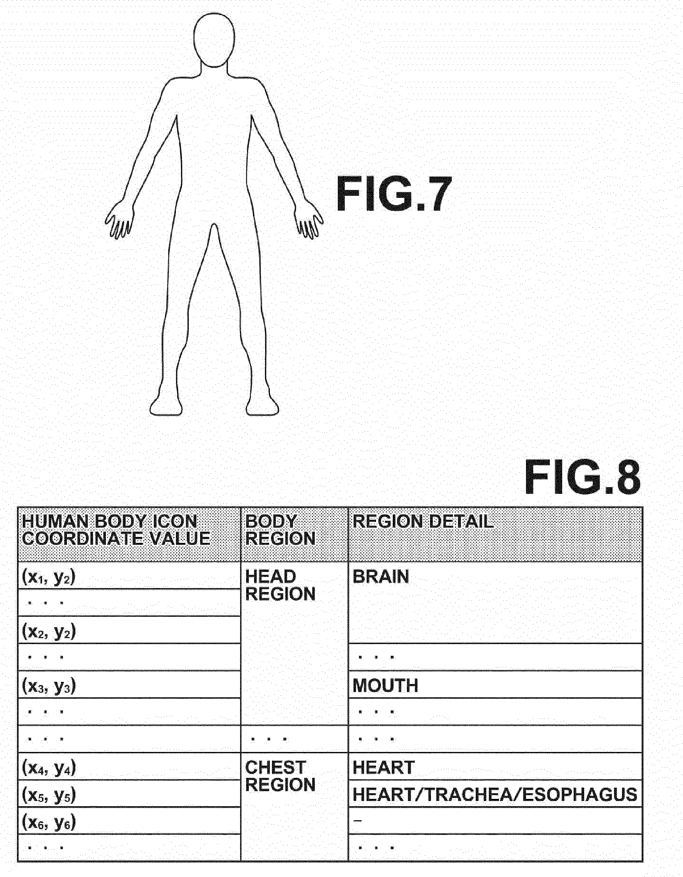

Next, obtaining condition input UI 32 reads a human body icon image (Figure 7) from the auxiliary storage device 17, displays the icon at a predetermined display position on the liquid crystal display 12 (#2), receives a gesture input of the user on the human body icon 45 from the touch panel 11, and stores the inputted gesture information in a predetermined area of the main memory 16 (#3). The human body icon 45 is a schematic representation of an entire human body, as illustrated, by way of example, in Figures 7 and 8, and region information for identifying a region of a body is related to each position of a coordinate system of the human body icon 45. The gesture information includes a touch type (start, movement, or end) at each time point, and position information representing a touched position in the coordinate system of the display device.

The gesture type analysis unit 33 determines to which of a plurality of predetermined gesture patterns the inputted gesture corresponds based on the gesture information and outputs a result of the determination to a predetermined area for storing gesture information in the main memory 16 (#4). If no gesture pattern corresponding to the inputted gesture is identified, the processing returns to a waiting state for input of a new gesture.

The gesture region analysis unit 34 first identifies on which position of the human body icon 45 the gesture input has been performed based on the gesture information and position information of the human body icon 45. That is, the gesture information is position information in the coordinate system of the display device, while the position information on the human body icon 45 is position information in the coordinate system of the human body icon 45, the gesture region analysis unit 34 converts the information of both positions to position information in the same coordinate system using information of display position of the human body icon 45 in the coordinate system of the display device. This allows a relative gesture position which is a point on the human body icon 45 constituting the gesture input to be identified. The relative gesture position may be represented by either the coordinate system of the display device or the coordinate system of the human body icon 45. In the present embodiment, it is represented by the coordinate system of the human body icon. Next, the gesture region analysis unit 34 identifies region information for identifying a region of the human body icon 45 related to the relative gesture position of the gesture and outputs the identified region information to a predetermined area for storing gesture region information in the main memory 16 (#5). If no region corresponding to the inputted gesture is identified on the human body icon 45, or if a gesture operation is performed only on the outside of the human body icon 45, the professing returns to a waiting state for input of a new gesture.

With reference to the obtaining condition table 46, the obtaining condition identification unit 35 identifies a medical information obtaining condition corresponding to the gesture type information outputted from the gesture type analysis unit 33 and the gesture region information outputted from the gesture region analysis unit 34, and outputs the identified medical information obtaining condition to a predetermined area of the main memory 16 (#6) . If no medical information obtaining condition corresponding to the inputted gesture is identified, the processing returns to a waiting state for input of a new gesture.

Next, the medical information obtaining unit 36 of the medical information display apparatus 1 transmits the medical information obtaining condition set by the obtaining condition identification unit 35 to the medical information management server 2 (#7). The medical information retrieval unit 52 of the medical information management server 2 receives the medical information obtaining condition from the medical information display apparatus 1 (#8), searches the medical information database 53 to extract real data of the medical information satisfying the received medical information obtaining condition (#9), and transmits the extracted real data of the medical information to medical information display apparatus 1 (#10). The medical information obtaining unit 36 of the medical information display apparatus 1 receives the transmitted real data of the medical information and stores them in a predetermined area of the main memory 16 or in the auxiliary storage device 17 (#11). Then, the medical information display control unit 37 displays the medical information on the liquid crystal display 12 based on the received real data of the medical information (#12). If no medical information satisfying the medical information obtaining condition is registered in the medical information database 53, information notifying accordingly is displayed.

As described above, in the first embodiment of the present invention, when a gesture performed on the human body icon 45 is inputted from the touch panel 11 of the medical information display apparatus 1, a medical information obtaining condition corresponding to the gesture is identified, then medical information satisfying the identified medical information obtaining condition is extracted from the medical information database 53 of the medical information management server 2, and the extracted medical information is displayed on the liquid crystal display 12 of the medical information display apparatus 1. Hereinafter, a series of processing steps performed until medical information corresponding to each of various gesture inputs is obtained will be described in detail.

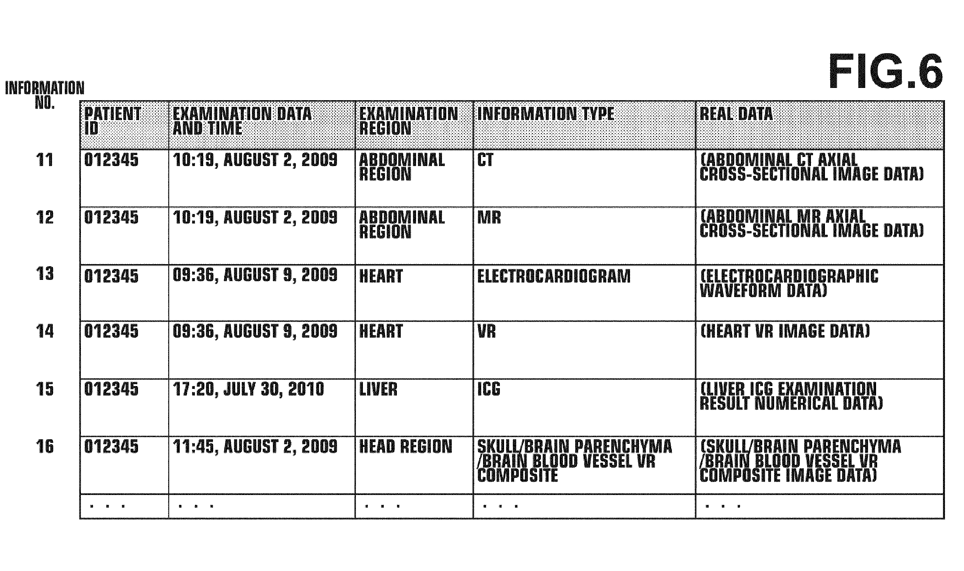

Figure 6 illustrates a data structure of the medical information database 53 and specific examples of medical information registered in the medical information database 53. As shown in the drawing, in the present embodiment, medical information database 53 includes patient ID, examination date and time, examination region, information type, and real data. The examination region and information type constitute index information corresponding to the medical information obtaining condition. Thus, the medical information obtaining condition identified by the obtaining condition identification unit 35 is constituted by an examination region condition representing a condition of examination region of the medical information to be obtained and an information type condition representing a condition of information type.

The medical information registration unit 51 collects medical information from each system and creates registration data to be registered in the medical information database 53 using the collected medical information. More specifically, a patient ID is extracted from each of the collected medical information and set to the patient ID entry of the medical information database 53. Information of a region of a patient is extracted from auxiliary information of each of the collected medical information or the like and set to the examination region entry. From auxiliary information of each of collected medical information or the like, a type of the information is extracted and set to the information type entry. Note that values based on a code system designed in advance are allocated to the examination region and information type. The examinations region and information type of each of the medical information is automatically set by the medical information registration unit 51 based on a predetermined setting condition. The collected real data are set to the real data entry. Then, the created registration data are registered in (inserted in) the medical information database 53. For example, in the case of axial cross-sectional image data, "abdominal region" is set to the examination region and "CT" is set to the information type, as the index information in the present embodiment (Information No. 11).

Figure 7 illustrates, by way of example, a human body icon 45. Figure 8 represents information included in the human body icon 45. As illustrated in Figure 8, region information for identifying a region of a human body is hierarchically related to each position of the coordinate system of the human body icon 45. That is, each position of the human body icon 45 is related to body region information representing a body region of a human body, such as a head region, a chest region, or the like, and region detail information representing a unit smaller than a region such as an internal organ, an organ, and the like. For example, the position of (x₁, y₁) of the human body icon belongs to the head region as the body region and belongs to the brain as the information detail. Further one position may be related to a plurality of sets of region detail information (heart, trachea, and esophagus, here), like the position (x₅, y₅) or it may be related to no region detail information, like the position (x₆, y₆).

In the present embodiment, the gesture type analysis unit 34 sequentially determines to which of four gesture patters of knife, specification, seizure, and hammer each inputted gesture corresponds.

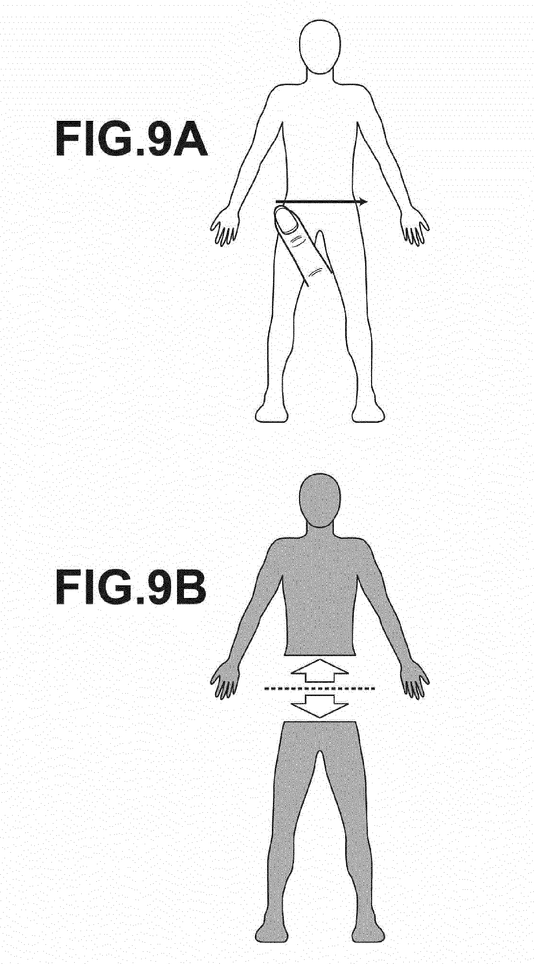

Figure 9A schematically illustrates, by way of example, a knife gesture. As illustrated in the drawing, a series of operations is performed by a user in which the user touches the right flank of the human body icon 45 on the touch panel 11 with a finger (start of touching), then moves the finger to the right direction with the finger kept touched (movement of touching), and removes the finger from the touch panel 11 at the left flank of the human body icon 45 (end of touching). This causes a gesture of slicing the abdominal region of the human body icon 45 to be received by the obtaining condition input UI 32.

If the trajectory of the inputted gesture is recognized as a straight line (line segment) by a known pattern recognition process based on position information at each time point from the start of touching, through movement of touching, to the end of touching included in the gesture information, the gesture type analysis unit 33 recognizes the gesture as a knife gesture and outputs gesture type information representing a knife gesture. Thus, the gesture in the example shown in Figure 9A is recognized as a knife gesture since the trajectory of the gesture is depicting a straight line.

If the gesture type information is a knife gesture, the gesture region analysis unit 34 identifies, based on the position information at each time point from the start of touching, through movement of touching, to the end of touching included in the gesture information, a relative position on the human body icon 45 at each time point by the coordinate conversion described above, and further obtains information of a body region and a region detail related to each identified relative position. In the example shown in Figure 9A, "abdominal region" is obtained as the region information and "large intestine", "small intestine", and the like are obtained as the region detail information.

If the inputted gesture is recognized as a knife gesture by the gesture type analysis unit 33, the obtaining condition input UI 32 may display the human body icon 45 separated along the trajectory of the gesture, as illustrated in the example shown in Figure 9B.

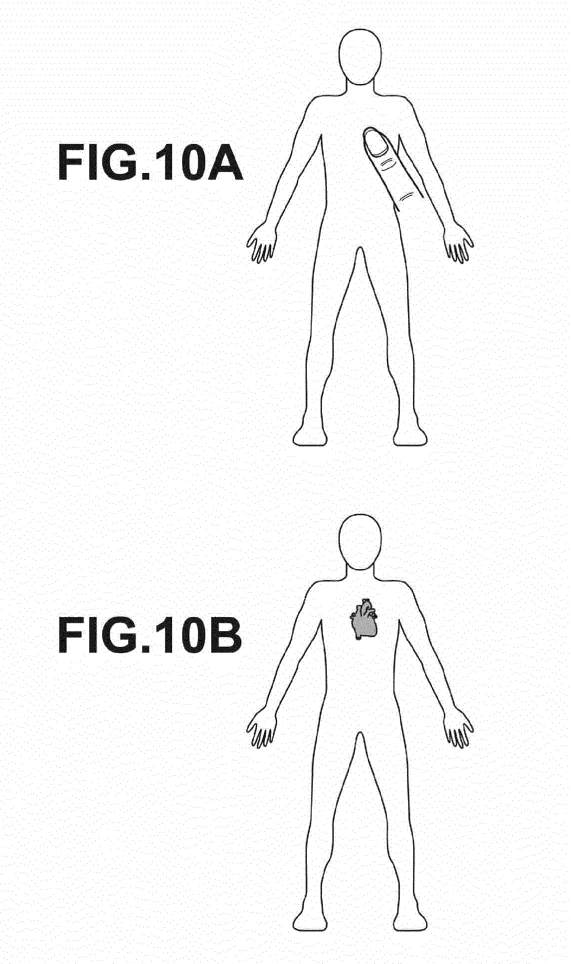

Figure 10A schematically illustrates, by way of example, a specification gesture. As illustrated in the drawing, a series of operations is performed by a user in which the user touches a position of the heart of the human body icon 45 with a finger (start of touching) and removes the finger from the touch panel 11 (end of touching) without moving the finger with the finger kept touched. This causes a gesture of tapping on the heart of the human icon 45 to be received by the obtaining condition input UI 32.

If the amount of movement of the touched position is determined to be smaller than a predetermined threshold value (close to zero) based on position information at each time point from the start of touching, through movement of touching, to the end of touching included in the gesture information, the gesture type analysis unit 33 recognizes the gesture as a specification gesture and outputs gesture type information representing a specification gesture. Thus, the gesture in the example shown in Figure 10A is recognized as a specification gesture since the amount of movement of the touched position is zero.

If the gesture type information is a specification gesture, the gesture region analysis unit 34 identifies, based on the position information at the start of touching or end of touching, a relative position on the human body icon 45 at the time by the coordinate conversion described above, and further obtains information of a body region and a region detail related to the identified relative gesture position. In the example shown in Figure 10A, "chest region" is obtained as the region information and "heart," and the like are obtained as the region detail information.

If the inputted gesture is recognized as a specification gesture by the gesture type analysis unit 33, the obtaining condition input UI 32 may display an organ or the like (heart, in this case) represented by the region detail information related to the specified position in a display mode different from that of the other areas of the human body icon 45, as illustrated in the example shown in Figure 10B. If the relative gesture position is related to a plurality of sets of region detail information (e.g., heart, trachea, and esophagus), all of them may be displayed in a display mode different from that of the other areas or having a user to determine which of them is to be displayed in a different display mode.

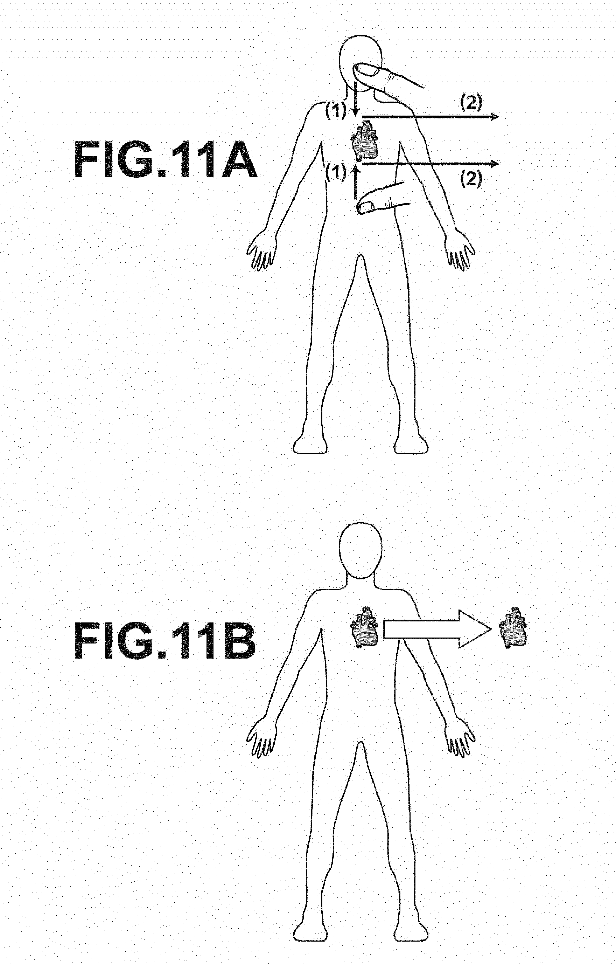

Figure 11A schematically illustrates, by way of example, a seize gesture. As illustrated in the drawing, this gesture uses a multi-touch gesture. First, a series of operations is performed by a user in which the user touches upper and down positions of the heart of the human body icon 45 with two fingers (start of touching, starting points of the arrows (1) in the drawing) and moves the fingers in the directions in which the heart is seized with the fingers kept touched (movement of touching, directions of the arrows (1) in the drawing), then further moves the fingers to the outside of the human body icon 45 with the fingers kept touched (movement of touching, the arrows (2) in the drawing), and removes the fingers from the touch panel 11 (end of touching, end points of the arrows (2) in the drawing) . This causes a gesture of seizing out the heart of the human body icon 45 is received by the obtaining condition input UI 32.

If a trajectory in which two touched points are moved in a first direction in which they come closer to each other and then the two points are moved in a second direction different from the first direction with the distance between them at the end of the movement in the first direction maintained is recognized by a known pattern recognition process based on position information at each time point from the start of touching, through movement of touching, to the end of touching included in the gesture information, the gesture type analysis unit 33 recognizes the gesture as a seize gesture and outputs gesture type information representing a seize gesture. Thus, the gesture in the example shown in Figure 11A is recognized as a seize gesture since the touched positions come closer to each other in the arrows (1) section and translated in a direction different from the arrows (1) in the arrows (2) section.

If the gesture type information is a seize gesture, the gesture region analysis unit 34 identifies, based on position information from the start of touching, through movement of touching, to the end of touching included in the gesture information, a position between two points at a time point at which the movement of the two touched points in the first direction ends and the movement direction is about to be changed (end point of each arrow (1) in Figure 11A) as a gesture position, then identifies a relative gesture position on the human body icon 45 by the coordinate conversion described above, and further obtains body region information and detailed body region information related to the identified relative gesture position. In the example shown in Figure 11A, "chest region" is obtained as the body region information at each position between end points of respective arrows (1) and "heart" and the like are obtained as the region detail information.

If the inputted gesture is recognized as a specification gesture by the gesture type analysis unit 33, the obtaining condition input UI 32 may display an organ or the like (heart, in this case) represented by the region detail information obtained by the gesture region analysis unit 34 in a display mode different from that of the other areas of the human body icon 45 and in an animated fashion in which the heart is seized out of the human body icon 45 by moving the heart in the second direction (arrows (2) direction), as illustrated in the example shown in Figure 11B. If the relative gesture position identified by the gesture region analysis unit 34 is related to a plurality of sets of region detail information (e.g., heart, trachea, and esophagus), all of them may be displayed in the manner described above or having the user to determine which of them is to be displayed in the manner described above.

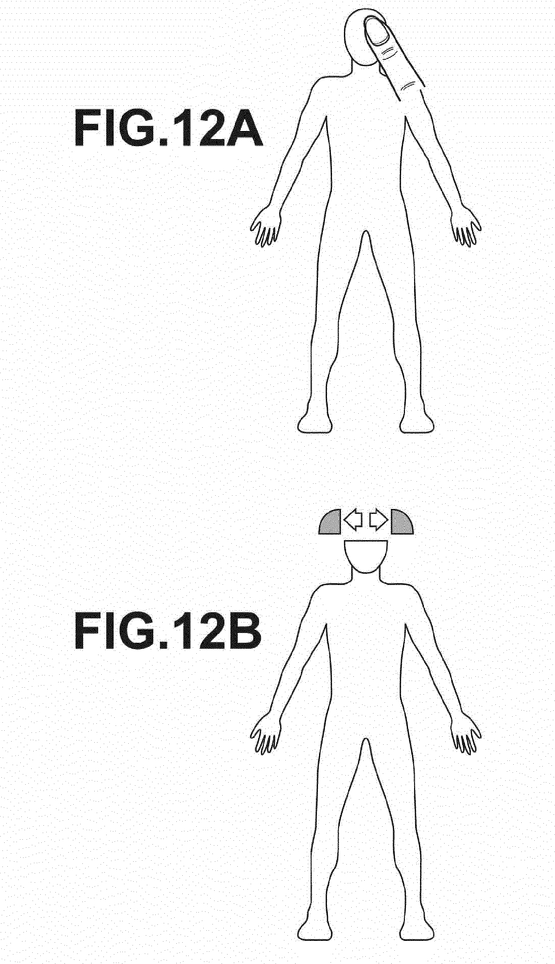

Figure 12A schematically illustrates, by way of example, a hammer gesture. As illustrated in the drawing, a series of operations is performed by a user in which the user touches a position of the head region of the human body icon 45 with a finger (start of touching), then keeps the finger touched on the position for over a predetermined time without moving the finger, and removes the finger from the touch panel 11 (end of touching). This causes a gesture of hammering and breaking the head region of the human icon 45 is received by the obtaining condition input UI 32.

If the amount of movement of the touched position is determined to be smaller than a predetermined threshold value (close to zero) and a gesture time from the start to end of touching is longer than a predetermined threshold value based on position information at each time point from the start of touching, through movement of touching, to the end of touching included in the gesture information, the gesture type analysis unit 33 recognizes the gesture as a hammer gesture and outputs gesture type information representing a hammer gesture. If the gesture time is shorter than the predetermined time, the gesture is recognized as a specification gesture in this recognition method. Thus, the gesture in the example shown in Figure 12A is recognized as a hammer gesture since the amount of movement of the touched position is zero and the gesture time is longer than the predetermined time.

If the gesture type information is a hammer gesture, the gesture region analysis unit 34 identifies, based on position information at the time point of the start or end of touching included in the gesture information, a relative gesture position on the human body icon 45 by the coordinate conversion described above, and further obtains body region information and detailed body region information related to the identified relative gesture position. In the example shown in Figure 12A, "head region" is obtained as the body region information and "brain" is obtained as the region detail information.

If the inputted gesture is recognized as a hammer gesture by the gesture type analysis unit 33, the obtaining condition input UI 32 may display an organ or the like (brain, in this case) represented by the region detail information related to the specified position in a display mode in which the brain appears to be broken down, as illustrated in the example shown in Figure 12B. If the relative gesture position is related to a plurality of sets of region detail information (e.g., heart, trachea, and esophagus), all of them may be displayed in a display mode different from that of the other areas or having a user to determine which of them is to be displayed in a different display mode.

For example, the gesture type analysis unit 33 may be configured to recognize an operation gesture of a predetermined medical instrument performed on the human body icon 45 (e.g., gesture of endoscope insertion operation) in addition to the gestures described above, and gesture region analysis unit 34 may be configured to recognize the operation target region of the medical instrument. Further, a gesture different from each gesture described above may be related to each gesture pattern described above.

As described above, the medical information obtaining condition identified by the obtaining condition identification unit 35 includes an examination region condition representing a condition with respect to examination region of the medical information to be obtained and an information type condition representing a condition with respect to information type. The obtaining condition identification unit 35 identifies an information type condition corresponding to the combination of gesture type information outputted from the gesture type analysis unit 33 and gesture region information outputted from the gesture region analysis unit 34 with reference to the obtaining condition table 46, and sets the gesture region information used to identify the information type condition to the examination region condition.

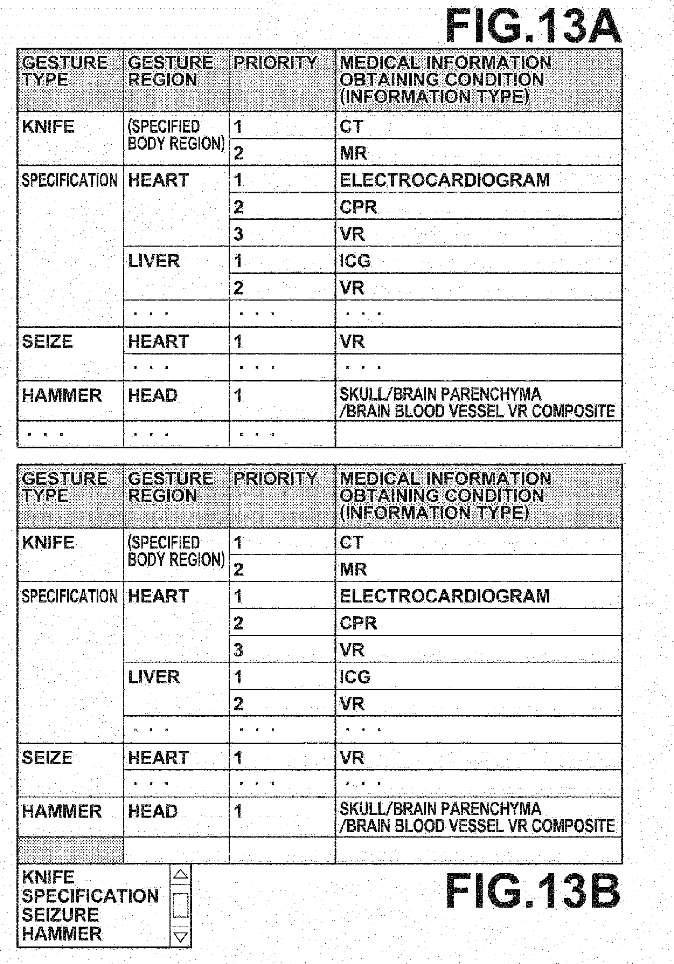

Figure 13A illustrates, by way of example, a setting of the obtaining condition table 46. As illustrated in the drawing, medical information obtaining conditions (information type conditions) are related to each combination of the gesture type and gesture region in a prioritized manner in the obtaining condition table 46 of the present embodiment.

The obtaining condition table 46 is editable by an obtaining condition table editing UI 38. Figure 13B illustrates, by way of example, an editing screen. As illustrated in the drawing, registered contents of the obtaining condition table 46 are displayed in a list form. In the case where the user wants to add a medical information obtaining condition, the user touches the last row of the list of each item to display a list box in which values that can be set to the item are listed. As the entry of gesture type is selected in Figure 13B, a list of gesture patterns that can be recognized by the gesture type analysis unit 33 is displayed in the list box. The user touches to select a desired value in the value list displayed in the list box and the selected value is set to the entry. In the case where a content of an existing medical information obtaining condition is to be changed, user touches an entry desired to be changed to display a list box similar to that described above, then the user touches to select a desired value, whereby the value of the entry is changed. In the case where an existing medical information obtaining condition is to be deleted, the user touches an arbitrary position in the row representing the medical information obtaining condition desired to be deleted with a finger, then the user moves the finger to the outside of the medical information obtaining condition list with the finger kept touched, and removes the finger from the touch panel, whereby the medical information obtaining condition is deleted.

In the case of the knife gesture performed on the abdominal region illustrated, by way of example, in Figure 9A, the obtaining condition identification unit 35 refers to the obtaining condition table 46 using all combinations of gesture type information and gesture region information, i.e., using each of the combination of gesture type information "knife" and region information "abdominal region" and the combination of gesture type information "knife" and region detail information "large intestine", "small intestine" and the like. As a result, it is known that the combination of gesture type information "knife" and body region information "abdominal region" corresponds to the combination of gesture type information "knife" and gesture region "specified body region(body region information outputted from the gesture region analysis unit 34)" in the obtaining condition table 46. Therefore, "CT", "mar" are obtained in the order of priority, as the information type conditions and "abdominal region" which is the gesture region information used for obtaining the aforementioned entry of the obtaining condition table 46 is obtained as the examination region condition.

The medical information retrieval unit 52 of the medical information management server 2 receives the medical information obtaining conditions identified by the obtaining condition identification unit 35 from the medical information obtaining unit 36 and retrieves the medical information database 53 using the received medical information obtaining conditions as the retrieval conditions in the order of priority. Note that, if medical information satisfying the current retrieval condition is extracted, database retrieval using a remaining lower priority medical information retrieval condition is not performed. On the other hand, if medical information satisfying the current retrieval condition is not extracted, database retrieval is performed with a medical information obtaining condition next higher in priority to the current retrieval condition as a new retrieval condition.

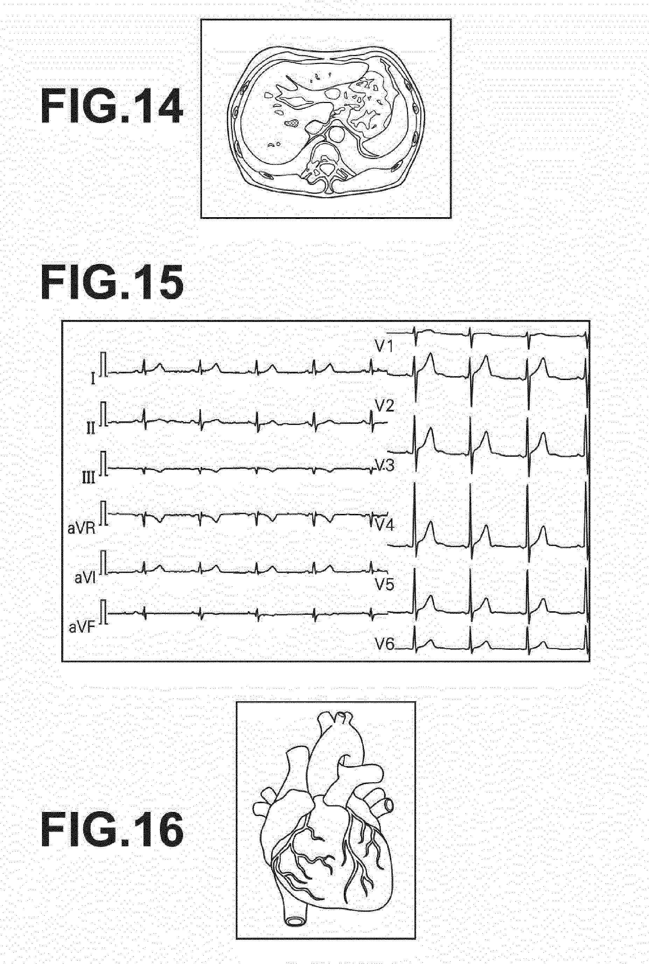

In the case of the knife gesture performed on the abdominal region illustrated, by way of example, in Figure 9A, the medical information retrieval unit 52 retrieves the medical information database illustrated, by way of example, in Figure 6 with the examination region condition as "abdominal region" and information type condition as "CT" having the highest priority (assuming here that "012345" is inputted as the patient ID) . The medical information retrieval unit 52 extracts CT axial cross-sectional image data, the real data of medical information in Information No. 11 that satisfies these conditions and the retrieval is completed. The extracted data are transmitted to the medical information obtaining unit 36 of the medical information display apparatus 1 and the medical information display control unit 37 displays a CT axial cross-sectional image like that shown in Figure 14. In the case where the medical information in Information No. 11 is not registered in the medical information database 53, no medical information corresponding to the retrieval condition described above is present in the database, so that the information type condition is changed to "MR" having a next higher priority and retrieval is performed again.

In the case of the specification gesture performed on the heart illustrated, byway of example, in Figure 10A, as a result of reference made by the obtaining condition identification unit 35 to the obtaining condition table 46 using all combinations of gesture type information and gesture region information, "electrocardiogram", "CPR", and "VR" which are information type conditions related to the gesture type "specification" and gesture region "heart" are obtained in the order of priority and "heart" which is the gesture region information used for obtaining the aforementioned entry of the obtaining condition table 46 is obtained as the examination region condition. Then, electrocardiographic waveform data in Information No. 13 corresponding to the retrieval condition with "heart" as the examination region condition and "electrocardiogram" as the information type condition are extracted by the medical information retrieval unit 52 of the medical information management server 2 and transmitted to the medical information obtaining unit 36 of the medical information display apparatus 1. The medical information display control unit 37 displays an electrocardiographic waveform like that shown in Figure 15.

In the case of the seize gesture performed on the heart illustrated, by way of example, in Figure 11A, as a result of reference made by the obtaining condition identification unit 35 to the obtaining condition table 46 using all combinations of gesture type information and gesture region information, "VR" which is the information type condition related to the gesture type "seize" and gesture region "heart" is obtained. As for the examination region condition, "heart" which is the gesture region information used for obtaining the aforementioned entry of the obtaining condition table 46 is obtained. Then, heart VR image data in Information No. 14 corresponding to the retrieval condition with "heart" as the examination region condition and "VR" as the information type condition are extracted by the medical information retrieval unit 52 of the medical information management server 2 and transmitted to the medical information obtaining unit 36 of the medical information display apparatus 1. The medical information display control unit 37 displays a VR image representing a heart like that shown in Figure 16.

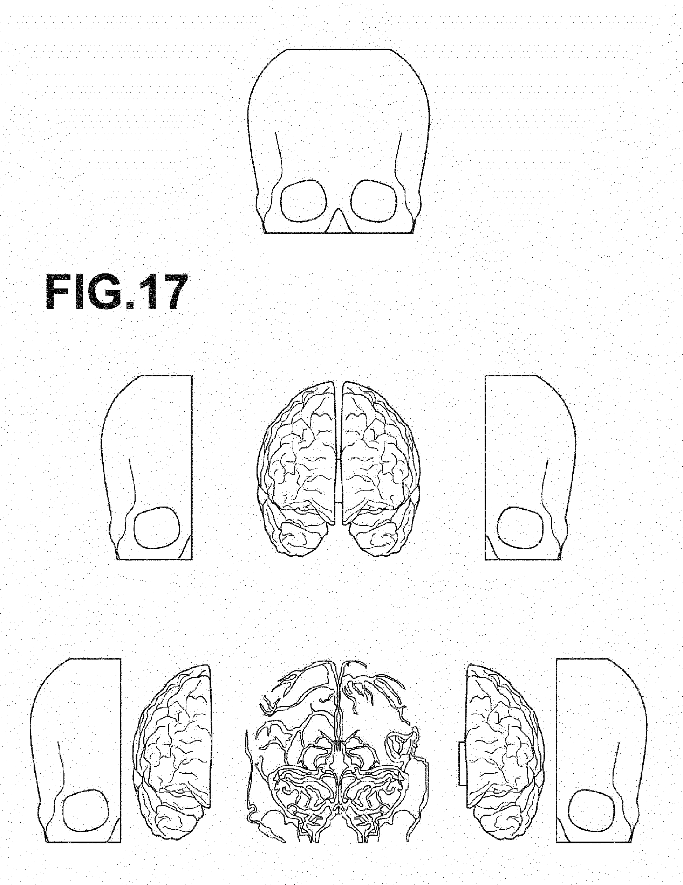

In the case of the hammer gesture performed on the head region illustrated, byway of example, in Figure 12A, as a result of reference made by the obtaining condition identification unit 35 to the obtaining condition table 46 using all combinations of gesture type information and gesture region information, "skull/brain parenchyma/brain blood vessel VR composite" which is the information type condition related to the gesture type "hammer" and gesture region "head region" is obtained. As for the examination region condition, "head region" which is the gesture region information used for obtaining the aforementioned entry of the obtaining condition table 46 is obtained. Then, skull/brain parenchyma/brain blood vessel VR composite image data in Information No. 16 corresponding to the retrieval condition with "head region" as the examination region condition and "skull/brain parenchyma/brain blood vessel VR composite" as the information type condition are extracted by the medical information retrieval unit 52 of the medical information management server 2 and transmitted to the medical information obtaining unit 36 of the medical information display apparatus 1. The medical information display control unit 37 displays a skull/brain parenohyma/brain blood vessel VR composite image like that shown in Figure 17. The skull/brain parenchyma/brain blood vessel VR composite image shown in Figure 17 may be obtained by performing volume rendering using volume data of non-contrast-enhanced head region CT and contrast-enhanced head region CT and combining obtained images. More specifically, a VR image of the entire skull may be obtained by performing volume rendering on the volume data of the non-contrast-enhanced head region CT based on color template and opacity curve for causing the CT value of the bone to become colored opacity. Likewise, a VR image of the entire brain parenchyma may be obtained by performing volume rendering on the volume data of the non-contrast-enhanced head region CT based on color template and opacity curve for causing the CT value of the brain parenchyma to become colored opacity. Further, left halves of the skull and brain parenchyma and right halves of the skull and brain parenchyma may be obtained by performing volume rendering similar to that described above on the volume data of non-contrast-enhanced CT representing left half and right half of the head region respectively. In the mean time, a VR image of the whole brain blood vessels may be obtained by performing volume rendering on the volume data of contrast-enhanced head region CT based on color template and opacity curve for causing the CT value of the contrast agent to become colored opacity. Finally, these VR images are combined so as to be arranged in the layout shown in Figure 17, whereby the skull/brain parenchyma/brain blood vessel VR composite image is obtained. Note that, a process in which the outer skull and the inner brain parenchyma of the head region are broken down may be displayed in an animated fashion by sequentially switching and displaying each of the VR image of the entire skull (top of Figure 17), the VR image of left and right skulls and VR image of the brain parenchyma (middle of Figure 17), and VR image of left and right skulls and brain parenchyma and the VR image of the whole brain blood vessels (bottom of Figure 17).

As described above, in the first embodiment of the present invention, different medical information may be obtained and displayed even for the same region of the human body icon 45 according to gesture patterns, such as, for example, the specification gesture performed on the heart shown in Figure 10A and seize gesture performed on the heart shown in Figure 11A.

As shown in the obtaining condition table 46 of Figure 13A, even for the same gesture, for example, specification gesture, if it is performed on the heart, electrocardiogram, CPR image of coronary artery, and VR image of heart will be obtained, while if it is performed on the liver, value of ICG examination result and VR image of liver will be obtained. In this way, even if the gesture patterns performed on the human body icon 45 are the same, different medical information may be obtained and displayed according to the region on which the gesture is performed.

As described above, in the first embodiment of the present invention, a gesture inputted through the obtaining condition input UI 32 is analyzed by the gesture type analysis unit 33 and gesture region analysis unit 34, whereby a gesture pattern and a position are obtained. Then, based on the obtained gesture pattern and position, a medical information obtaining condition intuitively represented by the gesture is identified by the obtaining condition identification unit 35. Then, medical information satisfying the identified medical information obtaining condition is obtained by the medical information obtaining unit 36 from the medical information database 53 of the medical information management server 2 and the obtained medical information is displayed on the liquid crystal display 12 by the medical information display control unit 37. Thus, the user may easily narrow down and obtain desired medical information for display only by a single action of performing an intuitive gesture on the touch panel 11 of the medical information display apparatus 1. In this way, the medical information display apparatus 1 of the present embodiment has extremely high operability and a high practical value.

As the obtaining condition table editing UI 38 for editing the obtaining condition table 46 is provided, it is possible to flexibly define medical information obtaining conditions that meet the requirements of clinical sites or preferences of users, whereby the operability and flexibility of the medical information display apparatus 1 may further be enhanced, thereby contributing to further improvement of working efficiency in the clinical sites.

Further, region information is hierarchically related to the human body icon 45, like the body region information and region detail information, so that it is possible to combine the gesture pattern and gesture region more flexibly and sophisticatedly.

The first embodiment described above is arranged such that, based on a single gesture from the start of touching, through the movement, to the end of touching, medical information corresponding to the gesture is obtained, that is, relatively simple and easy operability is provided in the first embodiment. In other words, a user interface for inexperienced beginners in operation is provided. On the other hand, this interface alone may possibly be insufficient in operability for skilled users. Consequently, in a second embodiment of the present invention, it is an object to provide a user interface having more complicated operability for skilled users in operation. A functional structure implemented in a medical information display apparatus and a medical information management server, and a flow of display processing performed in a medical information integration system in the second embodiment of the present invention are identical to those of the first embodiment (Figures 4 and 5).

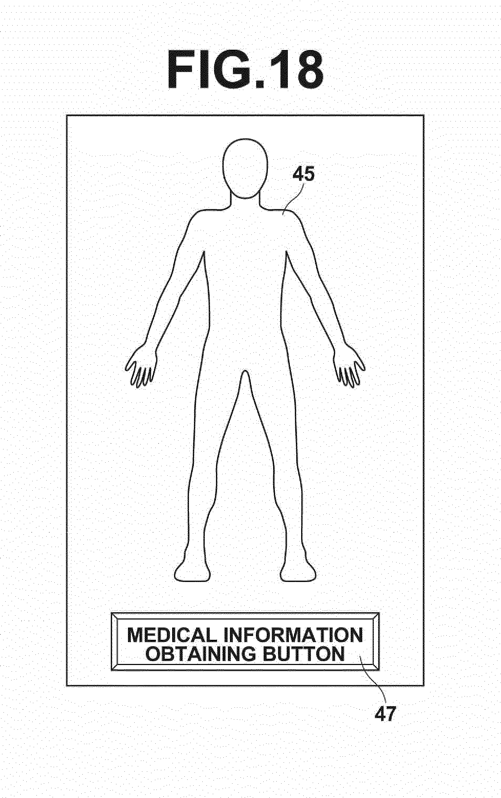

Figure 18 illustrates, by way of example, the obtaining condition input UI 32 in the second embodiment of the present invention. As illustrated in the drawing, a medical information obtaining button 47 is provided other than the human body icon 45. After a gesture operation on the human body icon 45 is completed, if the user touches the medical information obtaining button 47, the obtaining condition identification unit 35 identifies a medical information obtaining condition corresponding to one or more gestures performed so far. That is, whereas, when one gesture operation (from the start to end of touching) is completed, subsequent processing steps are performed automatically in the first embodiment, the medical information obtaining button 47 serves as a trigger for causing subsequent processing steps to be performed in the second embodiment. In the second embodiment, this allows a plurality of gesture operations on the human body icon 45 to be received.

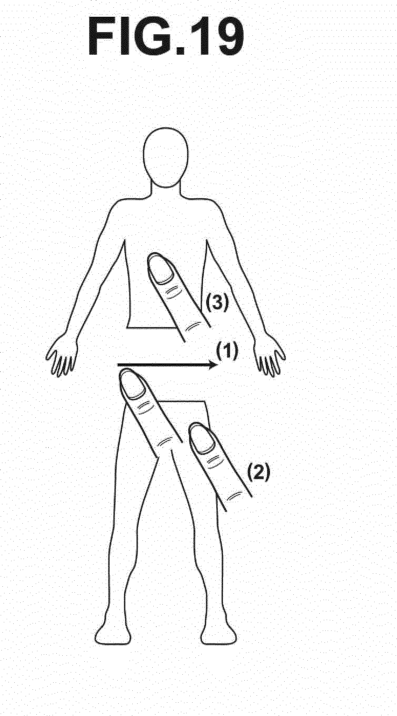

Figure 19 schematically illustrates, by way of example, input of obtaining condition combining a plurality of gesture operations. As indicated by (1) in the drawing, the user performs a knife gesture in the left to right direction on the abdominal region of the human body icon 45. In response to the knife gesture, the obtaining condition input UI 32 separates the human body icon 45 along the trajectory of the gesture and displays the separated icon. Next, as indicated by (2) and (3) in the drawing, the user performs specification gestures on the lower and upper sides of the separated human body icon 45 in this order.

The gesture type analysis unit 33 and the gesture region analysis unit 34 outputs gesture type information and gesture region information with respect to each of a plurality of gestures inputted through the obtaining condition input UI 32, that is, the gestures are divided by a unit from the start to end of touching, and the gesture type information and gesture region information are outputted with respect to each divided gesture.

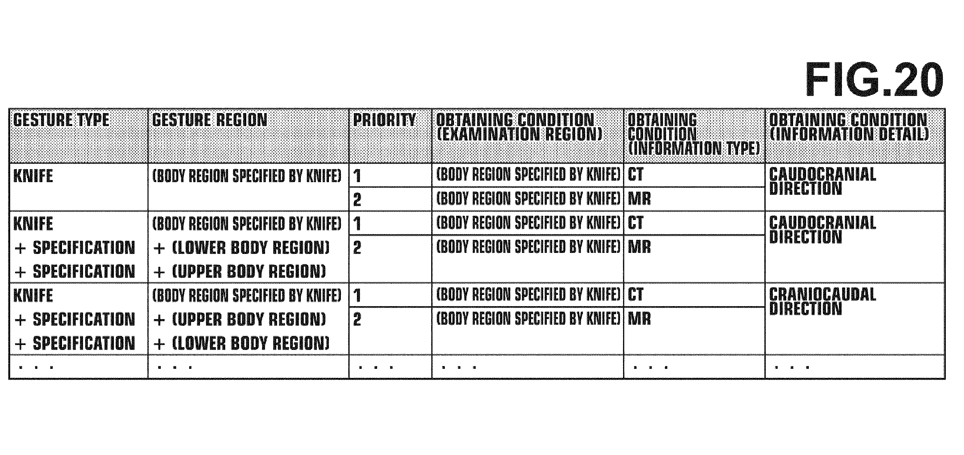

Figure 20 illustrates, by way of example, an obtaining condition table 46 corresponding to such input of a plurality of gestures. In the present embodiment, for an obtaining condition input operation that includes a plurality of gestures, a list of each gesture type and each gesture region arranged in the order in which the plurality of gestures is performed is registered in the table, as illustrated in the drawing. Further, in the present embodiment, not only the information type condition but also examination region condition and information detail condition are related to the combination of the gesture type and gesture region. In the case of the input example illustrated, by way of example, in Figure 19, a knife gesture performed on the abdominal region, a specification gesture specifying the lower side of the abdominal region, and a specification gesture specifying the upper side of the abdominal region are performed, so that the input example corresponds to the second combination of the gesture type and gesture region of the obtaining condition table 46 illustrated, by way of example, in Figure 20. Therefore, the examination region condition "body region specified by knife", i.e., "abdominal region", the information type condition "CT", "MR", and the information detail condition "caudocranial direction", which are medical information obtaining conditions corresponding to the aforementioned combination, are identified by the obtaining condition identification unit 35. In the input example illustrated, by way of example, in Figure 19, if the gestures of (2) and (3) are performed in the reverse order, the input example corresponds to the third combination of the gesture type and gesture region of the obtaining condition table 46 illustrated, by way of example, in Figure 20. Therefore, the examination region condition "body region specified by knife (abdominal region) ", the information type condition "CT", "MR", and information detail condition "craniocaudal direction" are identified. In this way, in the input example illustrated, by way of example, in Figure 19, the information detail condition is identified according to the order of disposition of the positions of the second and third specification gestures.

In the case of only the knife gesture illustrated, by way of example, in Figure 9A, it corresponds to the first combination of the gesture type and gesture region of the obtaining condition table 46 illustrated, by way of example, in Figure 20, and "body region specified by knife (abdominal region)", the information type condition "CT", "MR", and information detail condition "caudocranial direction" are identified. In this way, in the present embodiment, an obtaining condition input of one gesture and an obtaining condition input of two or more gestures may be received by maintaining the obtaining condition input period until the medical information obtaining button 47 shown in Figure 18 is touched.

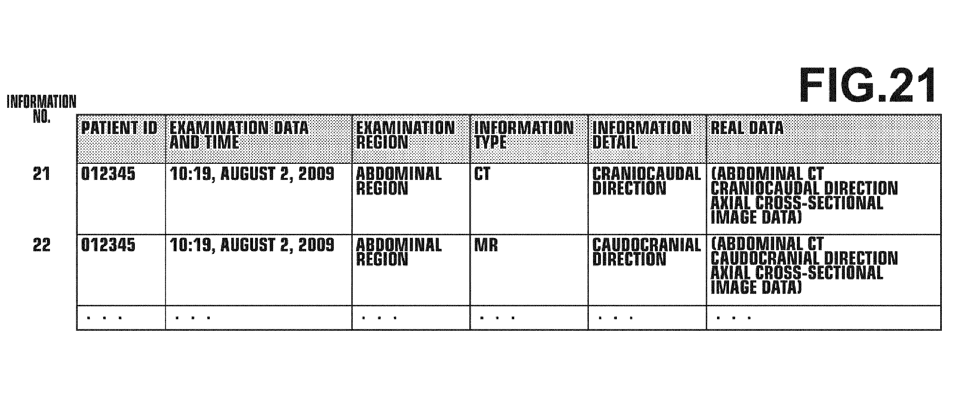

Figure 21 illustrates a data structure of the medical information database in the second embodiment of the present invention and an example of registered medical information corresponding to the example shown in Figures 19 and 20. As illustrated in the drawing, the data structure of the medical information database in the second embodiment further includes an entry of information detail as index information corresponding to the medical information obtaining condition in comparison with the medical information database of the first embodiment (Figure 6). Information detail extracted from auxiliary information of medical information and the like and serving as a complement of the information type of the medical information is set to the information detail entry by the medical information registration unit 51.

In the case of the input example illustrated, by way of example, in Figure 19, abdominal CT caudocranial direction axial cross-sectional image data in Information No. 22 that satisfies the examination region condition "abdominal region", information type condition "CT", and information detail condition "caudocranial direction" identified by the obtaining condition identification unit 35 are extracted from the medical information database 53 by the medical information retrieval unit 52 and transmitted to the medical information obtaining unit 36. The term "abdominal CT caudocranial direction axial cross-sectional image" as used herein refers to an axial cross-sectional image obtained from the abdominal CT and representing a cross-section viewed in an upward direction from the bottom of the patient (caudocranial direction). If the inputs of (2) and (3) illustrated in Figure 19 are performed in the reverse order, abdominal CT craniocaudal direction axial cross-sectional image data in Information No. 21 that satisfies the examination region condition "abdominal region", information type condition "CT", and information detail condition "craniocaudal direction" are extracted from the medical information database 53 by the medical information retrieval unit 52.

As described above, in the second embodiment of the present invention, the obtaining condition input UI 32 is capable of receiving an input which includes a plurality of gestures and more complicated than that in the first embodiment. Then, with respect to each of a plurality of inputted gestures, the gesture type and gesture region are recognized by the gesture type analysis unit 33 and gesture region analysis unit 34, and a medical information obtaining condition related to the types and regions of the plurality of gestures is identified by the obtaining condition identification unit 35. This allows an input of more information to be received by the obtaining condition input UI 32 and a more detailed medical information obtaining condition to be identified according to the amount of information.

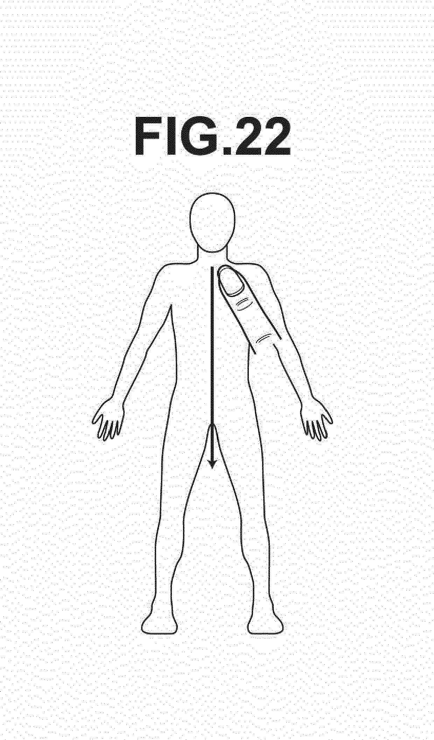

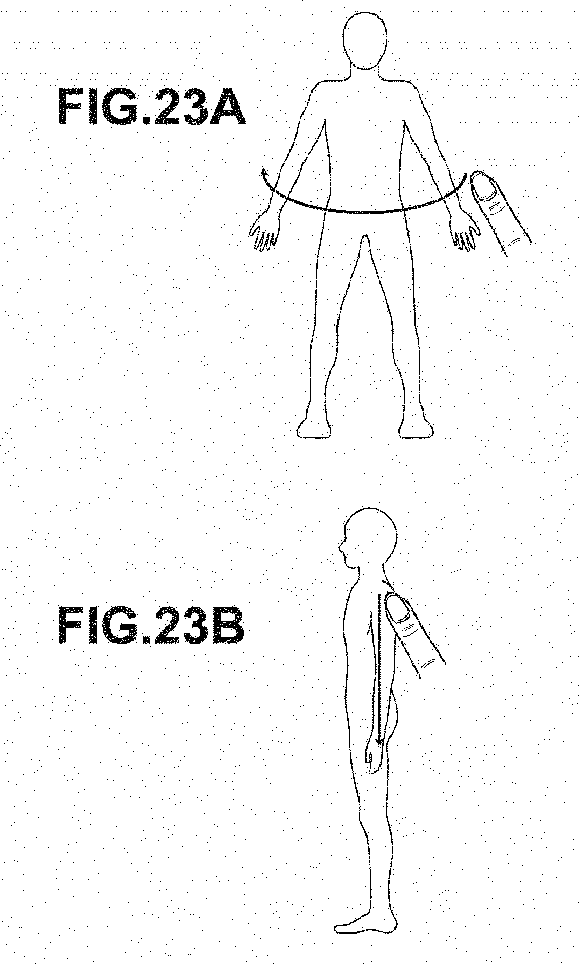

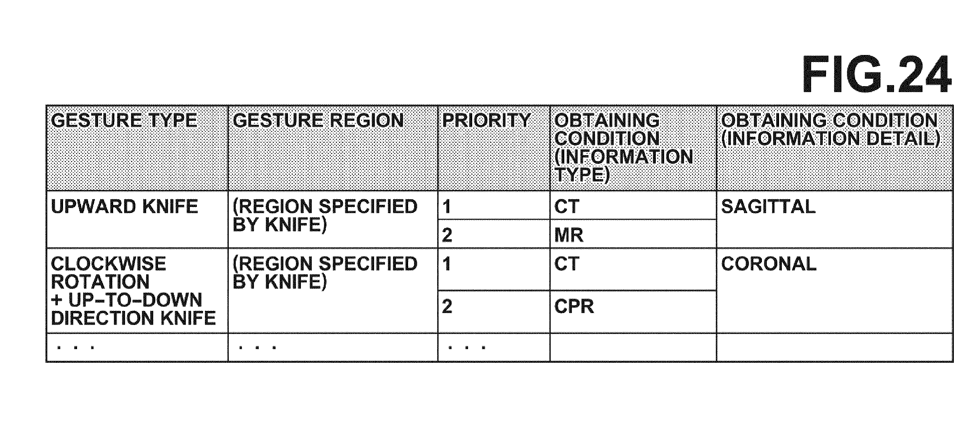

Figures 22 to 25 are provided for explaining other specific examples of the second embodiment of the present invention. Figure 22 illustrates an input example of medical information obtaining condition by a single gesture. As illustrated in the drawing, when a knife gesture is performed by the user in an up-to-down direction from the chest region to abdominal region of the human body icon 45, the gesture type analysis unit 33 in this specific example also recognizes the direction of the knife gesture and outputs "up-to-down direction knife" as the gesture type information. In the mean time, Figures 23A and 24B illustrate an input example of medical information obtaining condition by two gestures. As illustrated in Figure 23A, when a series of operations is performed by the user in which the user touches the left-hand side of the human body icon 45 with a finger, then moves the finger to the left side in an arc with the finger kept touched, and removes the finger at the right-hand side of the human body icon 45, gesture type analysis unit 33 in this specific example performs pattern recognition on the trajectory of the gesture and determines it as a gesture representing a rotation in a clockwise direction viewed from the head side of the human body icon 45. In response to the determination result, the obtaining condition input UI 32 rotates the human body icon 45 in the aforementioned clockwise direction by 90 degrees so as to be displayed like that shown in Figure 23B. Here, when a knife gesture is performed by the user in an up-to-down direction from the chest region to abdominal region of the human body icon 45, the gesture type analysis unit 33 outputs "up-to-down direction knife" as the gesture type information as in the specific example of Figure 22.

Figure 24 shows an obtaining condition table 46 corresponding to the specific examples. In the case of the input example of Figure 22, the obtaining condition identification unit 35 identifies "CT", "MR" as the information type conditions and "sagittal" as the information detail condition based on the first entry corresponding to the gesture type information "up-to-down direction knife" and the gesture region information "chest region/abdominal region". In the mean time, in the case of the input example of Figures 23A, 23B, the obtaining condition identification unit 35 identifies "CT", "MR" as the information type conditions and "coronal" as the information detail condition based on the second entry corresponding to the gesture type information "clockwise rotation + up-to-down direction knife" and the gesture region information "chest region/abdominal region". Note that the "chest region/abdominal region" which is the region information used to identify the medical information obtaining condition is set as the examination region condition in each example described above as in the first embodiment. Further, an entry of orientation of the human body icon may be added to the obtaining condition table 46 and, for example, "frontal" is set in the first entry while "lateral" is set in the second entry. In this case, the obtaining condition input UI 32 maybe configured to further output information representing the orientation of the human body icon 46.

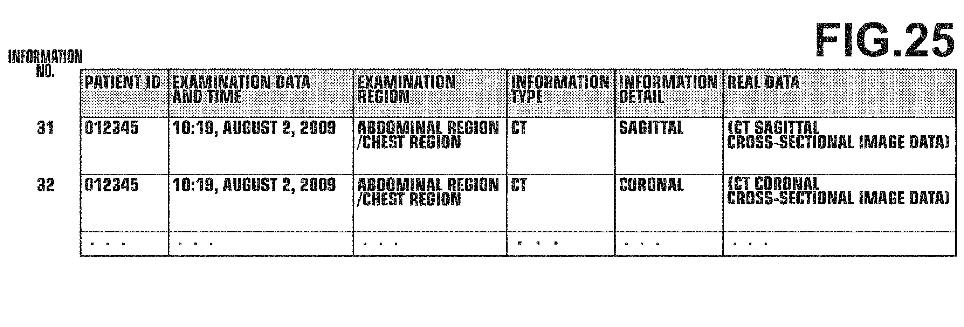

Figure 25 illustrates, by way of example, medical information registered in the medical information database corresponding to the specific examples. In the case of the input example of Figure 22, CT sagittal cross-sectional image data ranging from the chest region to abdominal region included in Information No.31 that satisfies the examination region condition "chest region/abdominal region", information type condition "CT", and information detail condition "sagittal" identified by the obtaining condition identification unit 35 are extracted from the medical information database 53 by the medical information retrieval unit 52 and transmitted to the medical information obtaining unit 36. In the mean time, in the case of the input example shown in Figures 23A, 23B, CT coronal cross-sectional image data ranging from the chest region to abdominal region included in Information No. 32 that satisfies the examination region condition "chest region/abdominal region", information type condition "CT", and information detail condition "coronal" identified by the obtaining condition identification unit 35 are extracted from the medical information database 53 by the medical information retrieval unit 52.

As described above, it is possible to identify the medical information obtaining condition for obtaining a cross-sectional image in the coronal direction by first rotating the human body icon 45 according to a rotation gesture, which is the first gesture performed through the obtaining condition input UI 32, and then receiving the up-to-down direction knife gesture which is the second gesture in the specific example.

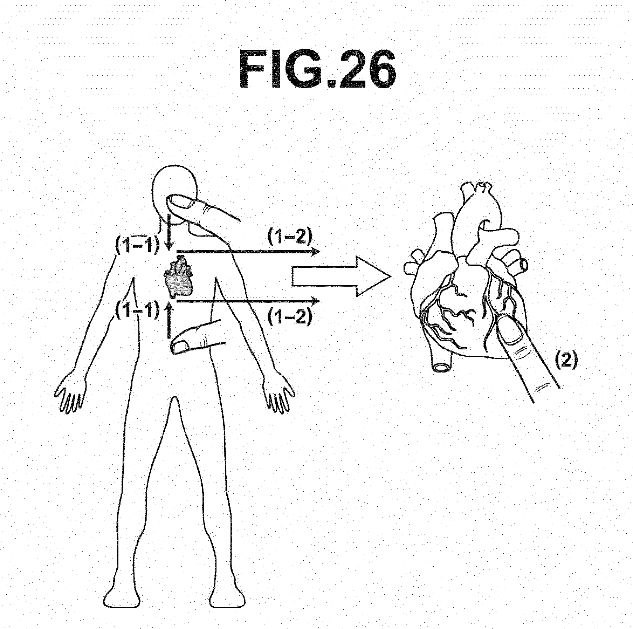

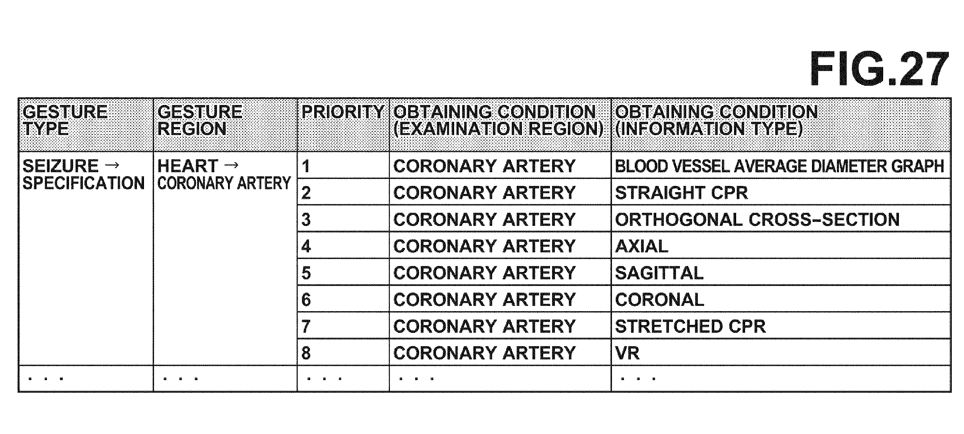

Figures 26, 27 illustrate still another specific example of the second embodiment of the present invention. Figure 26 illustrates a gesture input example in this specific example. As illustrated by (1-1) and (1-2) in the drawing, when a seize gesture is performed on the heart of the human body icon 45 by the user (Figure 11A), the gesture type analysis unit 33 recognizes the input gesture as a seize gesture, and the gesture region analysis unit 34 recognizes that the detailed target region of the gesture is the heart. In this specific example, the obtaining condition input UI 32 displays the heart, which is the seize gesture target, in a manner so as to be seized out of the human body icon 45 (Figure 11B) and displays an icon schematically representing a shape of the heart according to these recognition results. The heart icon is an icon in which each position is related to region information representing each region (coronary artery, left ventricle, and the like) of the heart as in the human body icon 45. As illustrated in (2) in the drawing, when a specification gesture is performed by the user on the position of coronary artery of the heart icon, gesture type analysis unit 33 recognizes the gesture as a specification gesture and the gesture region analysis unit 34 recognizes the gesture region as "coronary artery" based on the aforementioned relationship of the heart icon.

Figure 27 shows an obtaining condition table 46 corresponding to the specific example. In the case of the input example of Figure 26, the obtaining condition identification unit 35 identifies "coronary artery" which is the examination region condition corresponding to the gesture type information "seizure → specification" and gesture region information "heart → coronary artery", and various information type conditions.

As described above, it is possible to cause the heart icon to be displayed in response to the heart seize gesture which is the first gesture performed through the obtaining condition input UI 32, and to identify the medical information obtaining condition for obtaining the medical information of the coronary artery in response to the coronary artery specification gesture which is the subsequently performed second gesture. Further, in response to a size gesture performed on a given organ in the human body icon 45, it is also possible to cause an icon representing the organ to be displayed and to identify the medical information obtaining condition according to the gesture performed on the icon of the organ, as in the specific example. Thus, even for a fine structure for which it is difficult to input a gesture, such as a specific component of an organ or the like, it is possible to input the gesture by displaying an icon of the organ or the like in an enlarged form in response to a first gesture performed on the human body icon and receiving a second gesture input performed on the icon of the organ or the like, thereby resulting in improved operability.

Note that the gesture pattern explained in the first embodiment may be formed of a plurality of gestures. For example, gesture type analysis unit 33 may be configured to recognize two tapping operations within a predetermine time period as a specification gesture and three tapping operations as a hammer gesture.

In the case of the first and second embodiments, a medical information obtaining condition is identified first by the obtaining condition identification unit 35 of the medical information display apparatus 1 by analyzing an inputted gesture and then the medical information is obtained from the medical information management server 2. This may result in a prolonged wait time for the user of the medical information display apparatus 1 from the completion of the gesture to the display of the medical information, whereby the operability may be degraded. A third embodiment of the present invention is to solve such a problem.

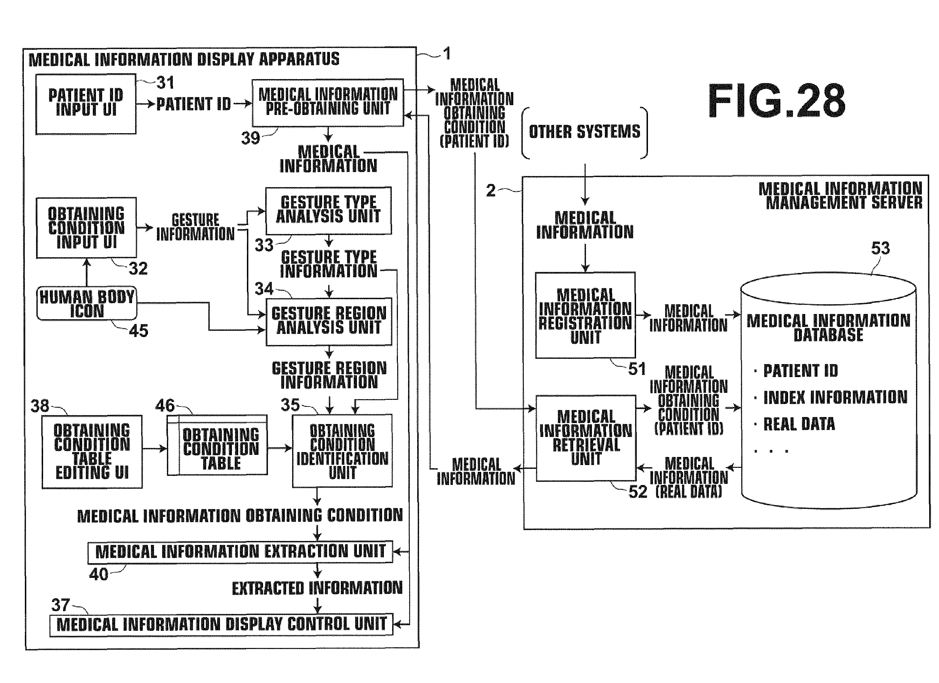

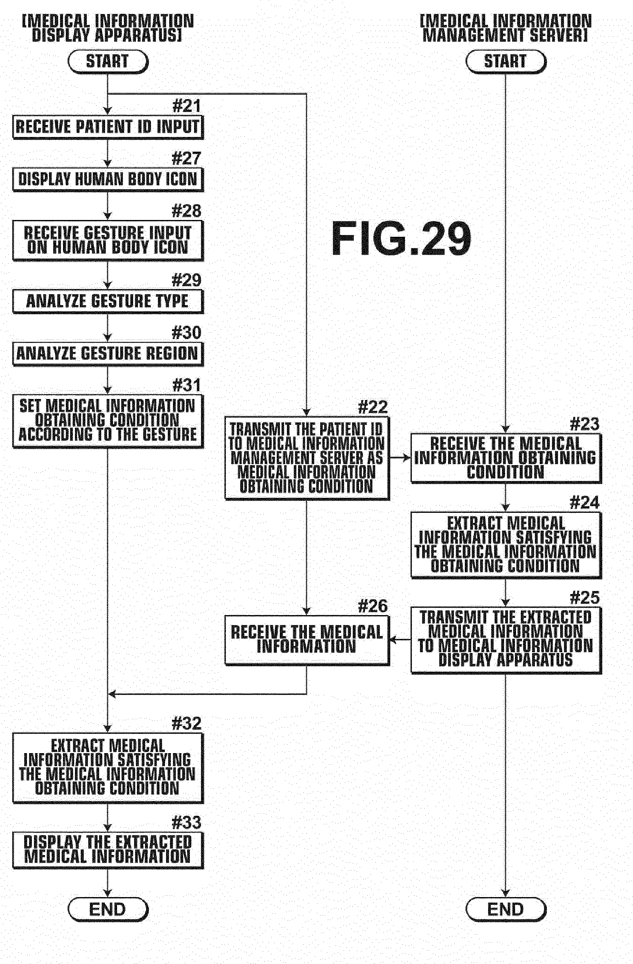

Figure 28 is a functional block diagram of a medical information display apparatus 1 and a medical information management server 2 in the third embodiment of the present invention. As illustrated in the drawing, the present embodiment is of a configuration in which a medical information pre-obtaining unit 39 is added to the medical information display apparatus 1 of the first and second embodiments and the medical information obtaining unit 36 is replaced by a medical information extraction unit 40. Figure 29 is a flowchart illustrating a processing flow for displaying medical information performed in the medical information integration system 3 in the third embodiment of the present invention.

As illustrated in the drawing, after an input of a patient ID is received by the patient ID input UI 31 as in the first embodiment (#21), the medical information pre-obtaining unit 39 transmits a medical information obtaining condition having only the inputted patient ID to the medical information management server 2 (#22). The medical information retrieval unit 52 of the medical information management server 2 receives the medical information obtaining condition (only the patient ID) from the medical information display apparatus 1 (#23), performs retrieval of the medical information database 53, and extracts medical information in the database whose patient ID corresponds to the patient ID of the received medical information obtaining condition (#24). Here, not only real data of the medical information but also index information corresponding to the medical information obtaining condition are extracted. The medical information retrieval unit 52 transmits the extracted medical information to the medical information display apparatus 1 (#25). The medical information pre-obtaining unit 39 of the medical information display apparatus 1 receives the transmitted medical information and stores the information in a predetermined area of the auxiliary storage device 17 or main memory 16 (#26).

In the mean time, the receiving of a gesture input and setting of a medical information obtaining condition according to the gesture are performed (#27 to #31) in the medical information display apparatus 1, as in the steps #2 to #7 of the first embodiment, while the aforementioned processing is performed by the medical information pre-obtaining unit 39.

Then, based on the medical information obtaining condition identified according to the inputted gesture and the index information included in the medical information obtained by the medical information pre-obtaining unit 39, the medical information extraction unit 40 extracts medical information that satisfies the identified medical information obtaining condition from the pre-obtained medical information (#32). Then, based on real data of the extracted medical information, the medical information display control unit 37 displays the medical information on the liquid crystal display 12 (#33).

As described above, in the third embodiment of the present invention, the medical information pre-obtaining unit 39 pre-obtains medical information related to the patient ID inputted through the patient ID input UI 31 from the medical information database 53 of the medical information management server 2 in parallel with the receiving of a gesture input by the obtaining condition input UI 32 and identification of a medical information obtaining condition by the obtaining condition identification unit 35 in the medical information display apparatus 1. When obtaining medical information that satisfies the medical information corresponding to the inputted gesture, this eliminates the need to gain access to the medical information database 53 of the medical information management server 2. This eliminates the need for the user of the medical information display apparatus 1 to wait for the retrieval operation performed by the medical information management server 2 and communication between the medical information display apparatus 1 and medical information management server 2, therefore, a throughput from the viewpoint of the user is improved and the operability is enhanced. Even when the medical information management server 2 and the network 9 have high loads or low performance, this embodiment may alleviate the influence thereof by pre-obtaining medical information.

In each of the aforementioned embodiments, the user is unable to know whether or not medical information with respect to the region on which a gesture is performed is present at the time when the gesture is inputted through the obtaining condition input UI 32. A fourth embodiment is to solve this problem.

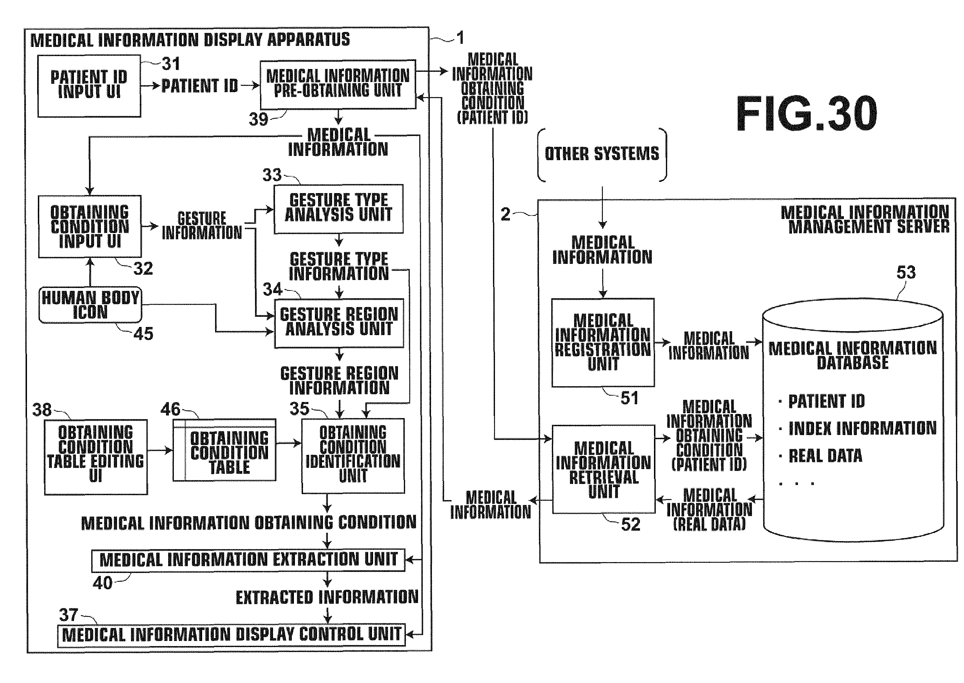

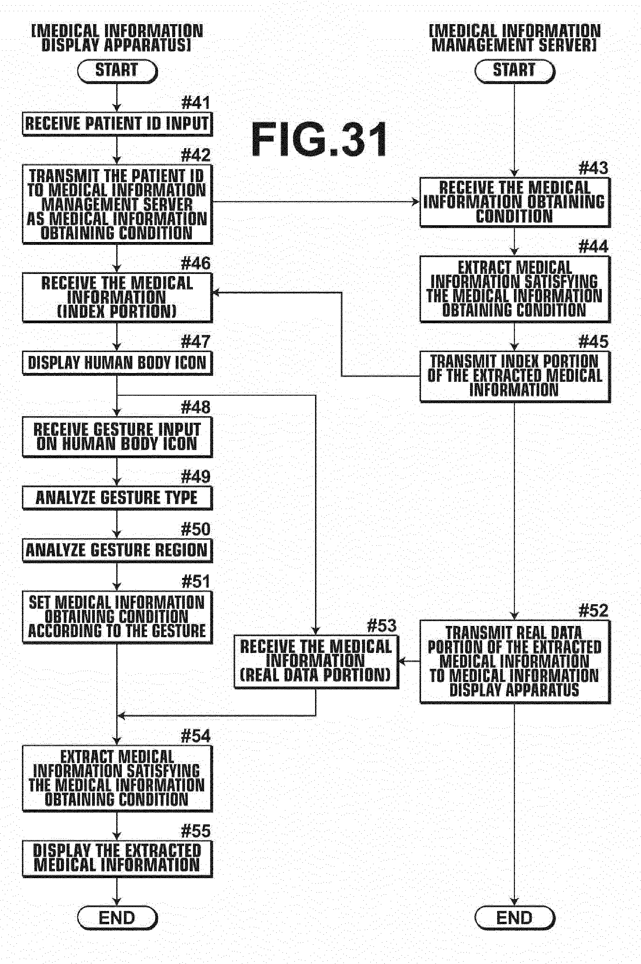

Figure 30 is a functional block diagram of a medical information display apparatus 1 and a medical information management server 2 in the fourth embodiment of the present invention. As illustrated in the drawing, the present embodiment is identical to the third embodiment in processing but differs from the third embodiment in that the obtaining condition input UI 32 uses medical information obtained by the medical information pre-obtaining unit 39. Figure 31 is a flowchart illustrating a processing flow for displaying medical information performed in the medical information integration system 3 in the fourth embodiment of the present invention.

As illustrated in the drawing, after an input of a patient ID is received by the patient ID input UI 31 as in the first embodiment (#41), the medical information pre-obtaining unit 39 transmits a medical information obtaining condition having only the inputted patient ID to the medical information management server 2 (#42) as in the third embodiment. The medical information retrieval unit 52 of the medical information management server 2 receives the medical information obtaining condition (only the patient ID) from the medical information display apparatus 1 (#43), performs retrieval of the medical information database 53, and extracts medical information in the database whose patient ID corresponds to the patient ID of the received medical information obtaining condition (#44). Here, not only real data of the medical information but also index information corresponding to the medical information obtaining condition are extracted. Now, in the present embodiment, the medical information retrieval unit 52 transmits only an index portion of the extracted medical information to the medical information display apparatus 1 (#45). The medical information pre-obtaining unit 39 of the medical information display apparatus 1 receives the transmitted index portion of the medical information and stores the information in a predetermined area of the auxiliary storage device 17 or main memory 16 (#46) . Further, in the present embodiment, while the aforementioned processing is performed by the medical information pre-obtaining unit 39, the display of a human body icon (step #27 in the third embodiment) is not performed.

The obtaining condition input UI 32 reads information of examination regions of the transmitted medical information, then classifies the regions in the human body icon into a group for which medical information is present and a group for which medical information is not present, and displays the human body icon 45 in a manner in which both groups are distinguishable (#47). Figure 30 illustrates an example human body icon to be displayed. In the example shown in the drawing, the regions for which medical information is present are indicated by a color darker than that of the regions for which medical information is not present. Then, as in each of the embodiments described above, the processing from the receiving of a gesture input on the human body icon by the obtaining condition input UI 32 (#48) to the identification of the medical information obtaining condition by the obtaining condition identification unit 35 (#51) is performed. While the processing described above is performed, the medical information retrieval unit 52 of the medical information management server 2 transmits real data of the medical information extracted in step #44 to the medical information display apparatus 1 (#52), and the medical information pre-obtaining unit 39 of the medical information display apparatus 1 receives the transmitted real data portion of the medical information, and stores the data in a predetermined area of the auxiliary storage device 17 or main memory 16 in the background (#43).

Then, as in the step #32 of the third embodiment, the medical information extraction unit 40 extracts medical information that satisfies the identified medical information obtaining condition from the pre-obtained medical information (#54), and medical information display control unit 37 displays the extracted medical information (#55).

As described above, in the fourth embodiment of the present invention, the obtaining condition input UI 32 displays the human body icon 45 in a manner in which a region for which medical information that can be displayed is present and a region for which medical information that can be displayed is not present are distinguishable. This allows the user to know whether or not medical information is present for each region of the human body icon 45 with respect to the patient specified by the patient ID input UI 31 before inputting a gesture through the obtaining condition input UI 32, whereby a redundant gesture input for which medical information can not be obtained may be avoided and the operation efficiency may be improved.

In order to display the human body icon 45 in the manner described above, it is necessary for the obtaining condition input 32 to refer to medical information pre-obtained by the medical information pre-obtaining unit 39. This makes it impossible to perform the display of the human body icon 45 and pre-obtaining of the entire medical information in parallel with each other, as in the third embodiment. Here, if the medical information retrieval unit 52 transmits the entire medical information extracted based on the patient ID, as in the third embodiment, the wait time from the entry of the patient ID to the display of the human body icon is increased, whereby the operability and working efficiency are degraded. Consequently, the medical information retrieval unit 52 is configured to transmit only the index portion of the extracted medical information required by the obtaining condition input UI 32 . This may largely reduce the wait time from the entry of the patient ID to the display of the human body icon in comparison with the case in which all items of medical information are received. Further, processing from the receiving of gesture input to identification of medical information obtaining condition and the processing of receiving real data of the medical information, which can be performed in parallel with each other, are performed in parallel, as in the third embodiment, so that the wait time from the completion of a gesture input to the display of desired medical information is reduced in comparison with the first and second embodiments.

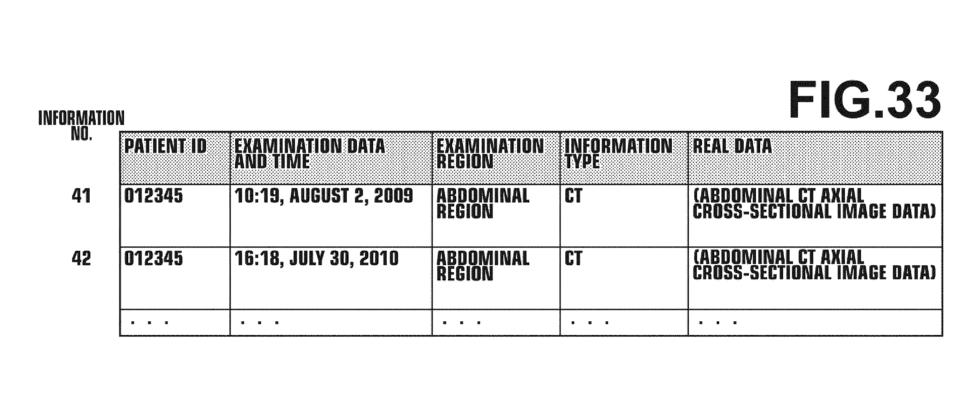

Each of the embodiments described above does not take into account the case in which sets of medical information of the same patient, same examination region, and the same information type with different examination dates and times are present, i.e., the case in which a plurality of sets of medical information satisfying medical information obtaining conditions of the same priority as, for example, in the registration example of the medical information database of Figure 33. In such a case, it is conceivable to make an arrangement in which medical information of the latest examination date and time is automatically selected or to provide a user interface for receiving selection of medical information to be displayed.

A fifth embodiment of the present invention is to realize a further effective display of medical information in such a case. A functional structure implemented in a medical information display apparatus and a medical information management server, and a flow of display processing performed in a medical information integration system in the fifth embodiment of the present invention are identical to those of each embodiment described above. Note that, however, if a plurality of sets of medical information is present, all of them are transmitted from the medical information management server 2 to the medical information display apparatus 1 and for the medical information to be transmitted, not only a real data portion but also an index portion are transmitted.

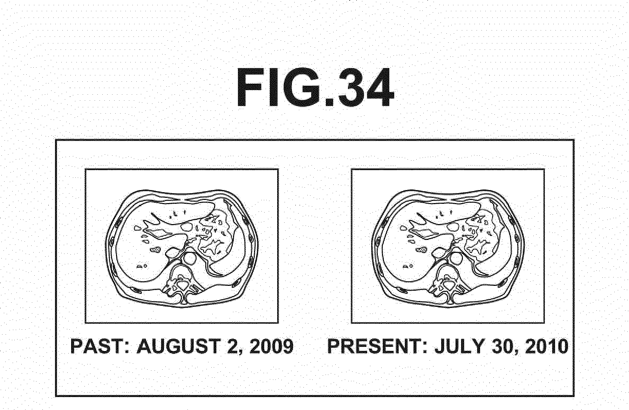

In the present embodiment, medical information display control unit 37 refers to the index portion of the display target medical information and, if sets of medical information of the same patient, the same examination region, and the same information type with different examination dates and times are present, displays them on the liquid crystal display 12 in a comparable manner. Figure 34 is a display example of two abdominal CT axial cross-sectional images of different examination dates and times shown in Figure 33 by way of example. As illustrated in the drawing, the two abdominal CT axial cross-sectional images are arranged side by side with examination dates attached thereto. This allows the user to easily compare a plurality of sets of medical information which differs only in the examination dates and times, whereby working efficiency is improved. Note that a user interface for receiving selection as to whether or not the aforementioned display is performed and if sets of medical information of the same patient, the same examination region, and the same information type with different examination dates and times are present, the aforementioned job may be performed according to the user selection.

In each of the embodiments described above, medical information having a higher priority is displayed based on the priority attached to the medical information obtaining condition. But there may be a case in which medical information having a lower priority corresponding to the inputted gesture is desired to be displayed. A sixth embodiment of the present embodiment takes into account such a case.

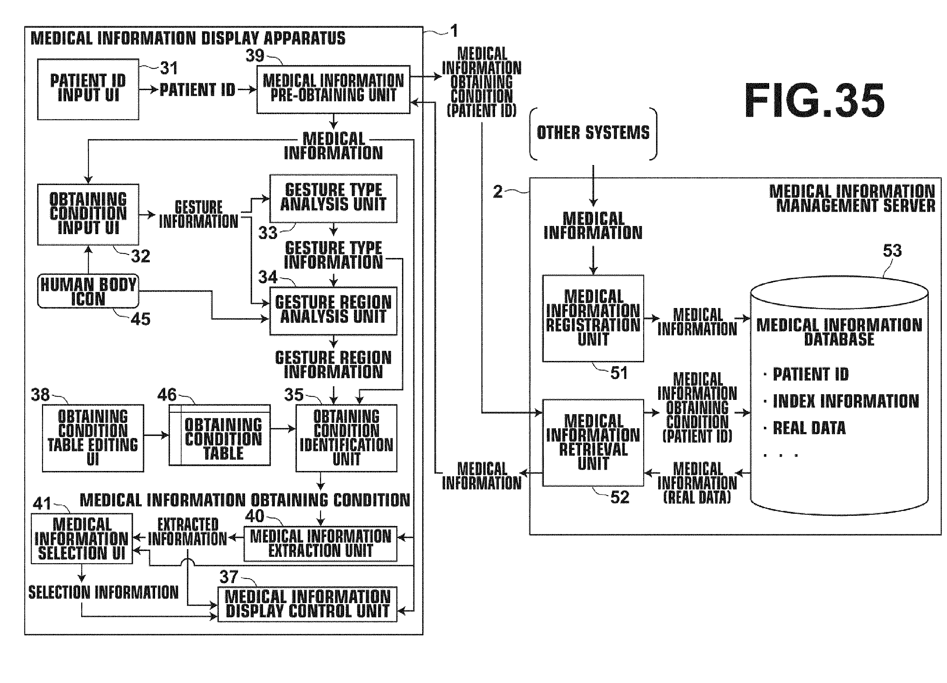

Figure 35 is a functional block diagram of a medical information display apparatus 1 and a medical information management server 2 in the sixth embodiment of the present invention. As illustrated in the drawing, the present embodiment is of a configuration in which a medical information selection UI 41 is added to the medical information display apparatus 1 of the fourth embodiment.

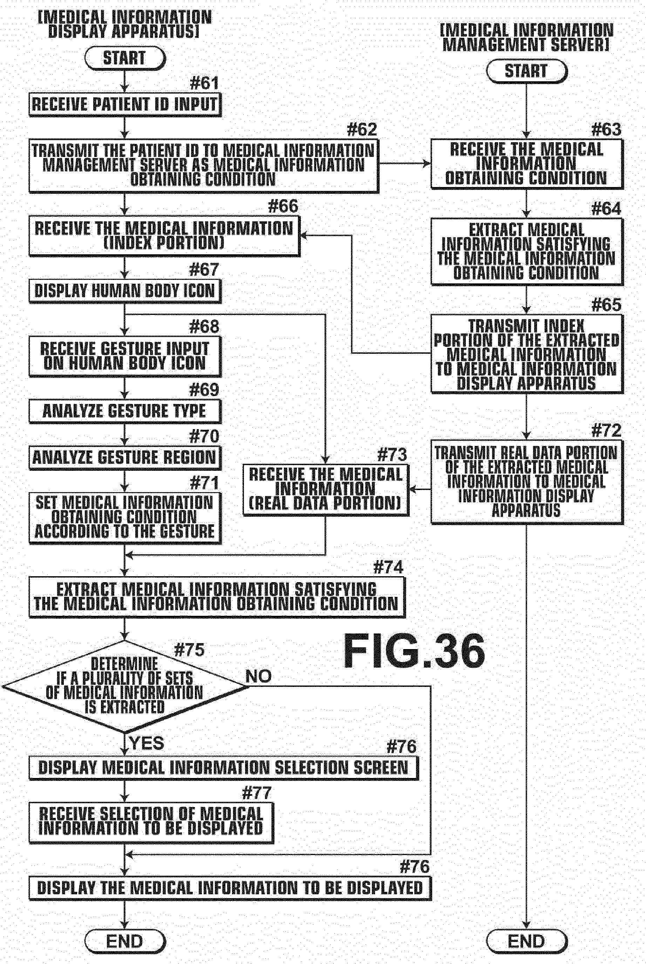

Figure 36 is a flowchart illustrating a processing flow for displaying medical information performed in the medical information integration system 3 in the sixth embodiment. As illustrated in the drawing, the steps #61 to #73 are identical to the steps #41 to #53 of the fourth embodiment.

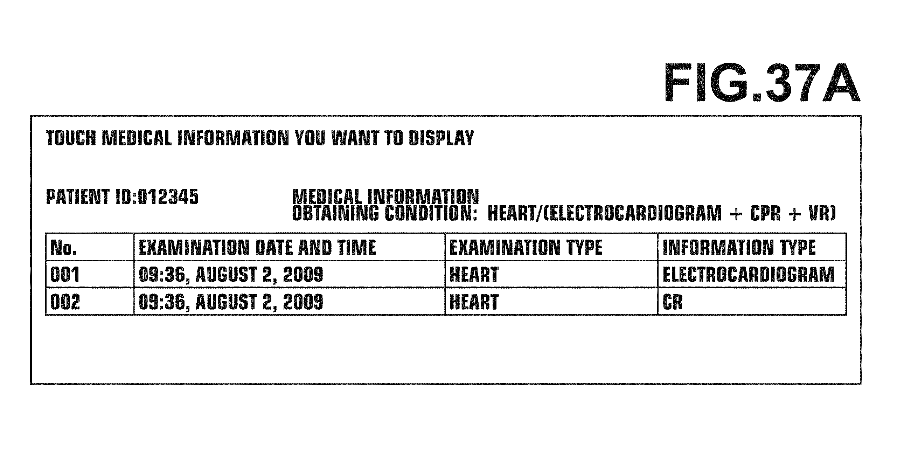

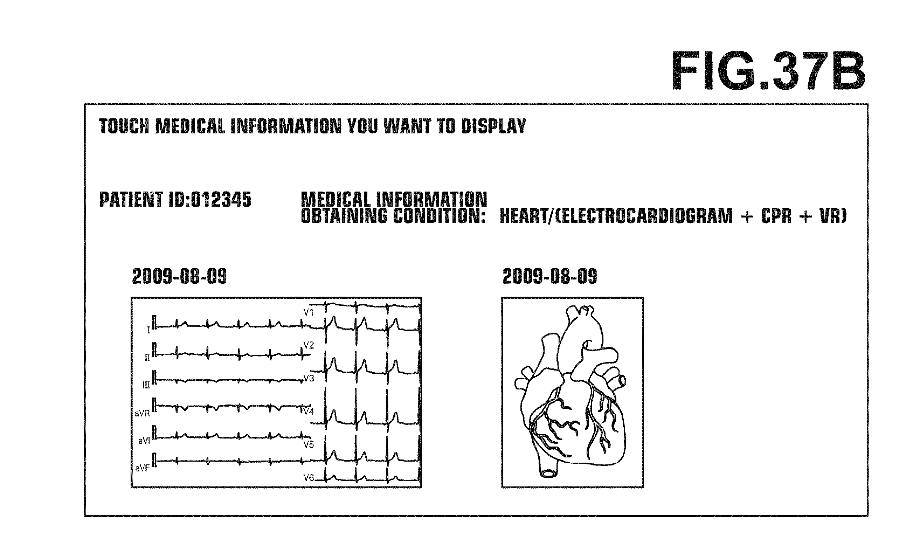

The medical information extraction unit 40 extracts medical information satisfying each of a plurality of medical information obtaining conditions of different priorities identified by the obtaining condition identification unit 35 (#74). Here, if one set of medical information is extracted by the medical information extraction unit 40 (#75; NO), the medical information display control unit 37 displays the medical information on the liquid crystal display 12 based on real data of the extracted medical information, as in the fourth embodiment described above (#78) . In the mean time, if a plurality of sets of medical information is extracted by the medical information extraction unit 40 (#75; YES), the medical information selection UI 41 displays a medical information selection screen in which extracted sets of medical information are listed in the order of priority of the medical information obtaining condition satisfied by each set of medical information on the liquid crystal display 12 (#76). Figure 37A illustrates, by way of example, a medical information selection screen. The screen is representing the case in which medical information corresponding to the specification gesture of heart illustrated, by way of example, in Figure 10A is extracted from the medical information database 53 illustrated, by way of example, in Figure 6. That is, the screen shows the case in which sets of medical information satisfying the patient ID = "012345", the examination region condition = "heart", and the information type condition = "electrocardiogram", "CPR", or "VR" are extracted by the medical information extraction unit 40. The medical information selection UI 41 displays a list in which index information portions of the extracted sets of medical information are arranged in the order of priority of the corresponding medical information obtaining conditions. Then, the medical information selection UI 41 receives a selection (touch operation) of the row representing the medical information desired by the user (#77). Figure 37B illustrates, by way of example, a visually more effective medical information selection screen. As illustrated in the drawing, sets of selection target medical information are displayed as thumbnails in the medical information selection screen. Here, for medical information for which an image interpretation report or an examination report is present, an icon representing the report may be attached to the thumbnail of the medical information related to the image interpretation report or examination report. Further, the thumbnails may be arranged in the order of priority corresponding to each set of medical information. Further, considering the case in which sets of medical information having the same priority with different examination date and time are extracted as in the fifth embodiment described above, the thumbnails may be arranged in the order of examination date and time or in the order of the combination of the priority and examination date and time. In this example screen, the medical information selection UI 41 receives a selection (touch operation) of a thumbnail image or an icon representing medical information desired by the user. Then, based on real data of the medical information selected by the medical information selection UI 41, the medical information display control unit 37 displays the medical information on the liquid crystal display 12 (#78).

As described above, in the sixth embodiment of the present invention, if a plurality of sets of medical information satisfying a medical information obtaining condition identified by the obtaining condition identification unit 35 is extracted by the medical information extraction unit 40 of the medical information display apparatus 1, the medical information selection UI 41 receives a selection of medical information to be displayed, so that the user may display desired medical information by a simple touch operation, whereby the operability is further enhanced.

For example, if the medical information is an image, each of the embodiments described above assumes that an image generated in the image diagnostic system 5 is registered in the medical information database 53. In contrast, a seventh embodiment of the present invention is to deal with the case in which volume data or the like are registered in the medical information database as medical information.

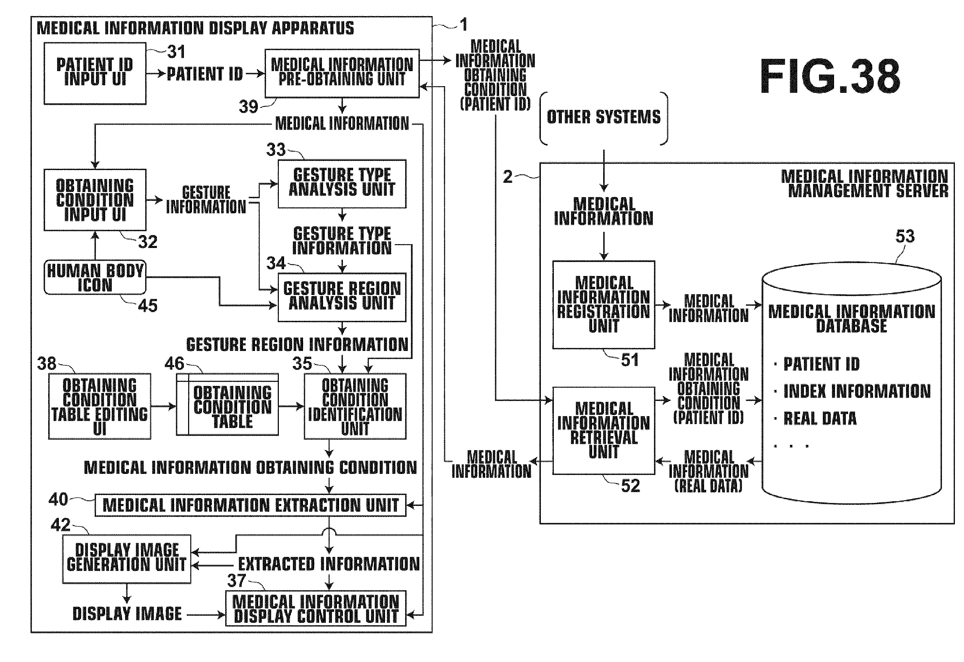

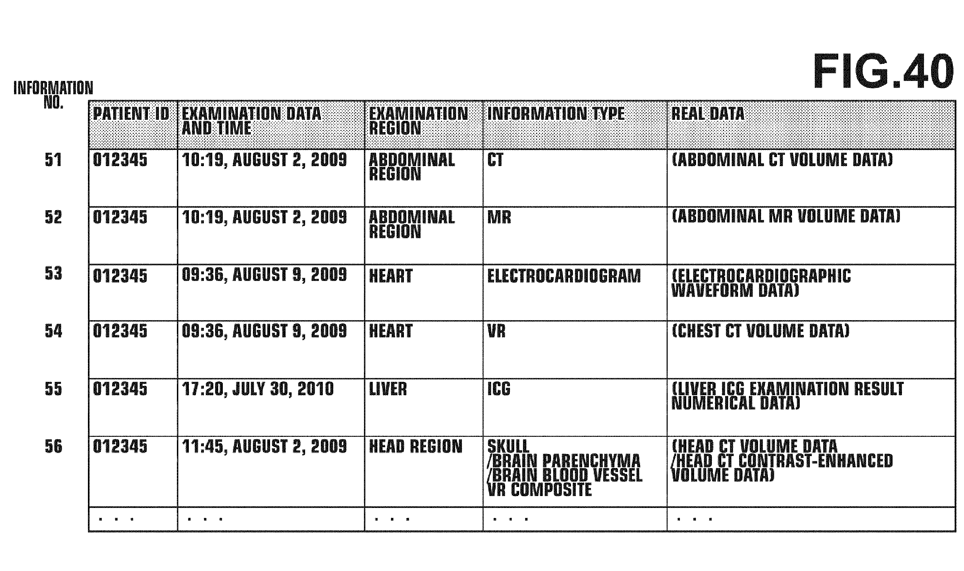

Figure 38 is a functional block diagram of a medical information display apparatus 1 and a medical information management server 2 in the seventh embodiment of the present invention. As illustrated in the drawing, the seventh embodiment is of a configuration in which a display image generation unit 42 is added to the medical information display apparatus 1 of the fourth embodiment. Further, medical information database 53 includes, in the data structure of the first embodiment, volume data obtained by various types of modalities, such as CT and the like, as real data of medical information, instead of a generated image, as specifically illustrated in Figure 40.

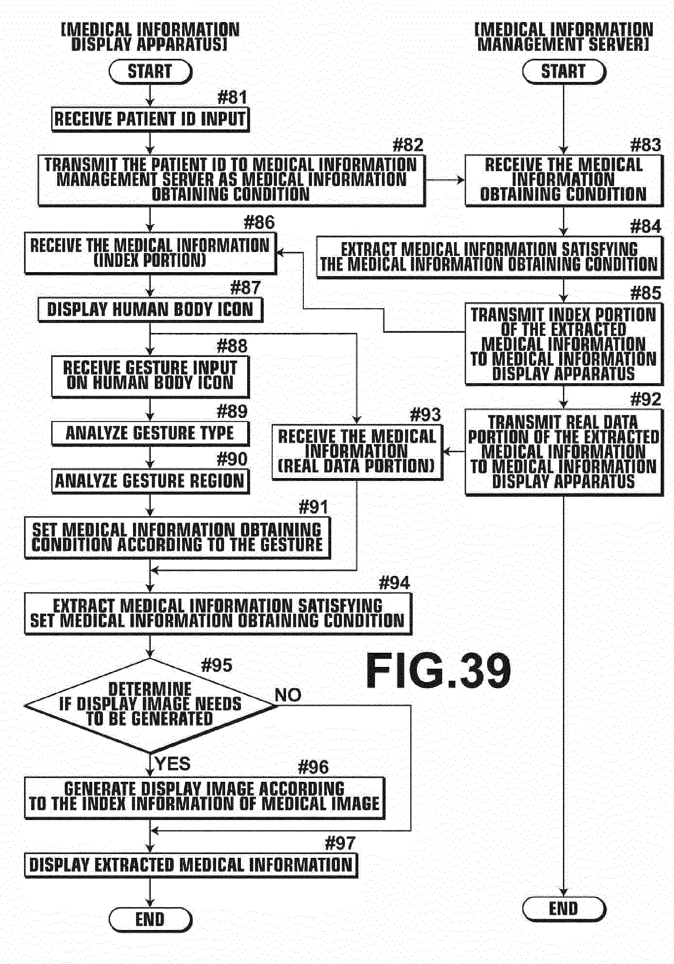

Figure 39 is a flowchart illustrating a processing flow for displaying medical information performed in the medical information integration system 3 in the seventh embodiment of the present invention. As illustrated in the drawing, the steps z81 to #94 are identical to the steps #41 to #54 of the fourth embodiment.

If volume data are extracted by the medical information extraction unit 40 as medical information satisfying the medical information obtaining condition(#95; YES), the display image generation unit 42 reads the examination region and information type of the medical information related to the volume data and generates an image according to the content thereof. For example, in the case of Information No. 51 of Figure 40, an axial cross-sectional image is generated from the abdominal CT volume data based on the examination region "abdominal region" and information type "CT". Here, the position of the axial cross-section may be a position predetermined by a startup parameter of the program, setting file, or the like. Otherwise, based on the position of a knife gesture (Figure 9A) received by the obtaining condition input UI 32, a ratio of the length from the top of the region (abdominal region) to which the position belongs to the position with respect to the length of the region in an up-to-down direction may be calculated and a position in a body axis direction in the extracted volume data corresponding to the ratio may be set as the position of the axial cross-section. If the extracted information, i.e., medical information in Information No. 51 is a collection of axial cross-sectional images, an axial cross-sectional image at the position determined by the aforementioned method may be selected from them. In the case of medical information in Information No. 54, a volume rendering image is generated from the chest CT volume data based on the examination region "heart" and information type "VR".

As described above, in the seventh embodiment of the present invention, instead of a generated medical image, volume data which are the original data of the image are registered in the medical information database 53 by the medical information registration unit 51, and if the medical information described above is extracted as the medical information satisfying the medical information obtaining condition, a display image is generated by the display image generation unit 42 according to the index information of the medical information. Thus, even in this case, medical information (image) identical to that of each embodiment may be displayed.

Further, in the present embodiment, if a user interface for changing an image generation condition is further provided, image generation conditions, such as the position and orientation of the cross-section, the color template and opacity curve of the volume rendering, the position of viewpoint and visual line direction, and the like, may be changed freely, and the display image generation unit 42 may generate a display image according to the changed image generation conditions. This allows more interactive medical information display to be realized in the medical information display apparatus 1.

In each of the aforementioned embodiments, a plurality of sets of medical information obtained based on medical information obtaining conditions of different priorities defined in the obtaining condition table 46 are not displayed simultaneously. There may be a case, however, in which these sets of medical information are required to be displayed at the same time depending on the user' s preference or demand from the medical sites. In such a case, it is a problem that in what layout a plurality of sets of medical information is to be displayed. An eighth embodiment of the present invention is to solve the problem.

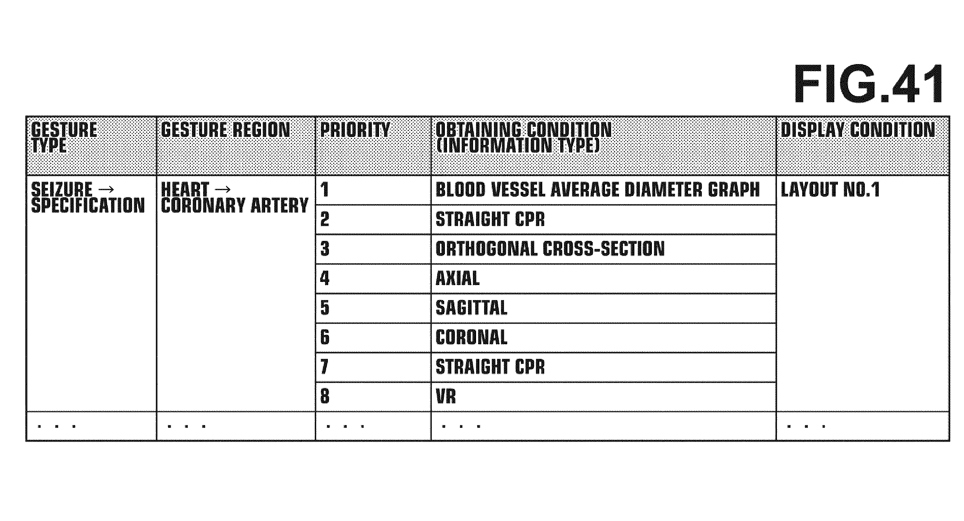

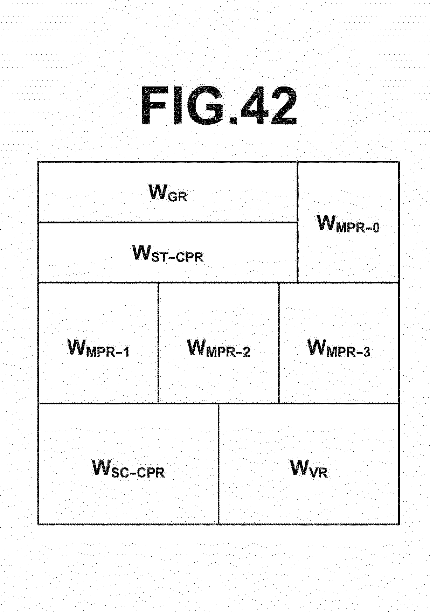

Figure 41 illustrates a structure of the obtaining condition table 46 in the eighth embodiment of the present invention. As illustrated in the drawing, the column of display condition is added. One display condition is defined for a plurality of sets of display target medical information. In the example in Figure 41, a display condition identified by the layout No.1 is related to the sets of medical information in Information No.1 to No.8. Figure 42 schematically illustrates, by way of example, a display condition of the layout No.1. As illustrated in the drawing, this display condition indicates a layout in which eight areas are arranged; namely, an area W_{GR} in which a graph GR representing average diameters of coronary arteries are displayed, an area W_{ST-CPR} in which a straight CPR image ST-CPR of coronary artery is displayed, an area W_{MPR-0} in which a MPR image MPR-0 representing an orthogonal cross-section of coronary artery is displayed, areas W_{MPR-1}, W_{MPR-2}, and W_{MPR-3} in which three MPR images MPR-1, MPR-2, andMPR-3 representing axial, sagittal, and coronal cross-sections are displayed respectively, an area W_{SC}-_{CPR} in which a stretched CPR image SC-CPR is displayed, and an area W_{VR} in which a volume rendering image VR is displayed.

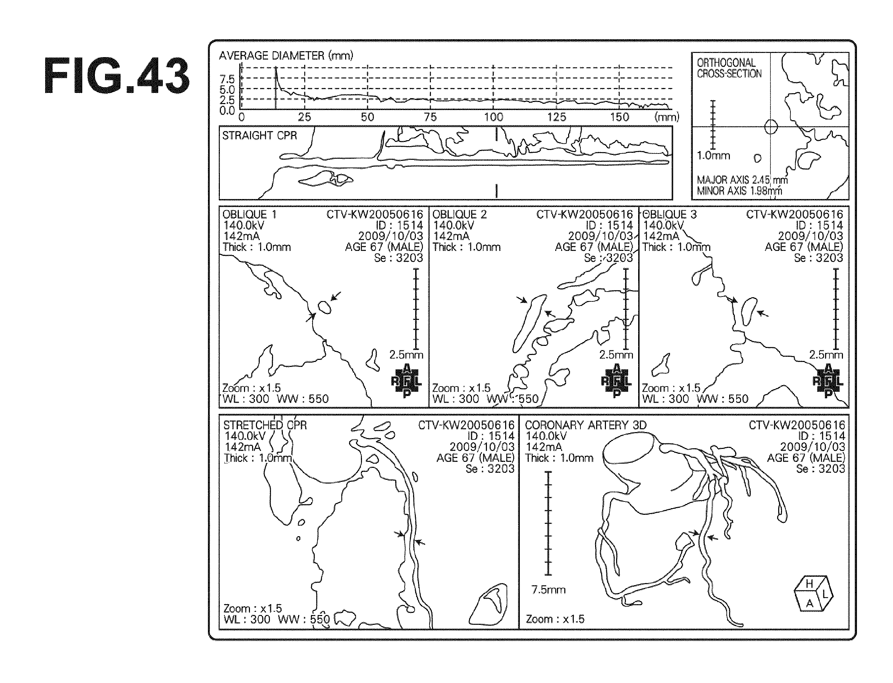

In the present embodiment, the medical information obtaining unit 36 or medical information extraction unit 40 obtains all sets of medical information, each satisfying each of medical information obtaining conditions of different priorities. Further, with reference to the obtaining condition table 46, the medical information display control unit 37 identifies the display condition related to the medical information obtaining condition that each set of the obtained medical information satisfies and displays each set of the obtained medical information based on the identified display condition. Figure 43 illustrates, by way of example, a display of each set of medical information satisfying each of the medical information obtaining conditions illustrated, by way of example, in Figure 41 based on the layout No.1 (Figure 42) related to each set of medical information.

As described above, in the present embodiment, medical information display control unit 37 may identify a display condition related to a medical information obtaining condition corresponding to display target medical information with reference to the obtaining condition table 46 and display the display target image information based on the identified display condition. Thus, in the case in which a plurality of sets of display target medical information is present, the sets of medical information may be displayed in an appropriate layout.

Each embodiment described above is provided for illustrative purposes only and all the explanations above should not be used to limit the technical scope of the present invention. Further, various changes and modifications made to the system configurations, hardware configurations, processing flows, module configurations, user interfaces, specific processing contents, and the like without departing from the spirit of the present invention are included in the technical scope of the present invention.

For example, a characteristic configuration of each embodiment may be combined, as appropriate, to produce a new embodiment. More specifically, the obtaining condition table 46 of the second embodiment of the present invention may be employed in the third to eighth embodiments, and the medical information selection UI 41 may be employed in the first and second embodiments.

Further, the description has been made of a case in which real data of medical information are also registered in the medical information database 53 of each embodiment. But, an arrangement may be adopted in which, instead of real data of medical information, link information (address information) for gaining access to the real data is registered in the database 53 and the real data stored in a database of the source system of the real data are to be used (by setting the hyperlink destination to the database of the source system of the data), and the real data may be obtained based on the link information only when the medical information becomes the display target.

In the embodiments described above, medical information management server 2 for integrally managing medical information is provided and medical information display apparatus 1 obtains medical information from the medical information database 53 of the medical information management server 2. But an arrangement may be adopted in which medical information is obtained directly from each of other systems, such as the image diagnostic system 5, the endoscopic examination system 6, and the like.

Further, the medical information display apparatus 1 may include the medical information database 53. In this case, it is only necessary to provide the function of the medical information retrieval unit 52 in the medical information obtaining unit 36 or medical information pre-obtaining unit 39.

In the embodiments described above, the description has been made of a case in which the medical information display apparatus 1 is a portable device, as illustrated in Figure 2, but it may be a desktop device having a liquid crystal display with a touch panel and a computer body.

Further, although the content of a gesture inputted from the touch panel 11 is analyzed by the gesture type analysis unit 33 and gesture region analysis unit 34, but an arrangement may be adopted in which whole or part of the analysis is performed by the operating system of the medical information display apparatus 1 or by a touch panel driver (software).

Still further, the image which is an example of medical information may be a moving image instead of a still image.

## Claims

1. A medical information display apparatus, comprising:
a display means for displaying given information;
a gesture input means for detecting a gesture operation performed on a display surface of the display means and outputting gesture information representing a content of the detected gesture operation;
a first display control means for displaying a subject appearance image representing an appearance of a subject at a predetermined display position of the display means based on image data of the subject appearance image, wherein each position of the image is related to region identification information for identifying a region of the subject;
a gesture type analysis means for determining, based on gesture information outputted according to a gesture operation detected by the gesture input means while the subject appearance image is displayed, a gesture type representing to which of a plurality of predetermined gesture operation patterns the detected gesture operation correspond;
a gesture region analysis means for identifying a gesture region which is a region of the subject corresponding to the detected gesture operation based on information of the display position of the subject appearance image, the region identification information related to the subject appearance image data, and gesture position information representing a position on which the gesture operation has been performed included in the gesture information outputted according to the gesture operationwhile the subject appearance image is displayed;
an obtaining condition identification means for identifying a medical information obtaining condition for obtaining medical information of the subject corresponding to the gesture operation performed while the subject appearance image is displayed based on the gesture type and the gesture region;
a medical information obtaining means for selectively obtaining medical information satisfying the identified medical information obtaining condition from a medical information storage means storing a plurality of sets of medical information; and
a second display control means for displaying the obtained medical information on the display means.

2. The medical information display apparatus of Claim 1, wherein the gesture input means is a touch panel type input means.

3. The medical information display apparatus of Claim 1 or 2, wherein the subject appearance image is an image schematically representing the subject.

4. The medical information display apparatus of any of Claims 1 to 3, wherein the first display control means is a means that displays the subject appearance image by changing the appearance of the subject in the subject appearance image to a predetermined appearance according to the detected gesture operation based on the gesture type and/or the gesture region corresponding to the detected gesture operation while the subject appearance image is displayed.

5. The medical information display apparatus of any of Claims 1 to 3, wherein:
the first display control means is a means that displays the subject appearance image by changing the appearance of the subject in the subject appearance image to a predetermined appearance according to the detected gesture operation based on a first gesture type determined by the gesture type determination means with respect to a first gesture operation detected while the subject appearance image is displayed and/or a first gesture region identified by the gesture region determination means with respect to the first gesture operation; and
the obtaining condition identification means is a means that identifies, based on at least some of the first gesture type, the first gesture region, a second gesture type determined by the gesture type determination means with respect to a second gesture operation detected while the changed subject appearance image is displayed, and a second gesture region identified by the gesture region determination means with respect to the second gesture operation, a medical information obtaining condition corresponding to the first and second gesture operations.

6. The medical information display apparatus of any of Claims 1 to 5, wherein the obtaining condition identification means is a means that identifies the medical information obtaining condition based on reference data in which a medical information obtaining condition is related to a combination of gesture type and gesture region.

7. The medical information display apparatus of Claim 6, wherein the reference data are data in which one or more medical information obtaining conditions are related to a pair of gesture type and gesture region and if two or more medical information obtaining conditions are related to the pair, a priority is further related to each of the plurality of medical information obtaining conditions.

8. The medical information display apparatus of Claim 6 or 7, wherein the apparatus further comprises a reference data editing means for editing the reference data.

9. The medical information display apparatus of any of Claims 1 to 8, wherein:
the apparatus further comprises a medical information pre-obtaining means for pre-obtaining medical information satisfying a given condition from the medical information storage means; and
the first display control means is a means that displays each region of the subject represented in the subject appearance image such that a region whose medical information is included in the pre-obtained medical information differs in appearance from a region whose medical information is not included in the pre-obtained medical information.

10. The medical information display apparatus of any of Claims 1 to 9, wherein the second display control means is a means that, when a plurality of sets of medical information with respect to examinations of the same type with different examination times is obtained by the medical information obtaining means, displays the plurality of sets of medical information in a comparable manner.

11. The medical information display apparatus of any of Claims 1 to 10, wherein the apparatus further comprises an image processing means for performing, when a medical image representing the subject is obtained by the medical information obtaining means from the medical information storage means, predetermined processing on the obtained medical image to obtain a medical image satisfying the medical information obtaining condition, as required.

12. The medical information display apparatus of any of Claims 1 to 11, wherein:
the apparatus further comprises a selection receiving means for list-displaying, when a plurality of sets of medical information satisfying the medical information obtaining condition is obtained by the medical information obtaining means, the plurality of sets of medical information on the display means and receiving selection of medical information to be displayed; and
the second display control means is a means that displays the medical information selected by the selection receiving means.

13. The medical information display apparatus of Claim 12, wherein the selection receiving means is a means that displays, when performing the list-display, the plurality of sets of medical information in the form of thumbnails or icons.

14. A medical information display system in which a medical information supply apparatus for selectively supplying medical information of a subject based on a given medical information obtaining condition and a medical information display apparatus for displaying the medical information are communicatively linked via a network, wherein the medical information display apparatus comprises:
a display means for displaying given information;
a gesture input means for detecting a gesture operation performed on a display surface of the display means and outputting gesture information representing a content of the detected gesture operation;
a first display control means for displaying a subject appearance image representing an appearance of a subject at a predetermined display position of the display means based on image data of the subject appearance image, wherein each position of the image is related to region identification information for identifying a region of the subject;
a gesture type analysis means for determining, based on gesture information outputted according to a gesture operation detected by the gesture input means while the subject appearance image is displayed, a gesture type representing to which of a plurality of predetermined gesture operation patterns the detected gesture operation correspond;
a gesture region analysis means for identifying a gesture region which is a region of the subject corresponding to the detected gesture operation based on information of the display position of the subject appearance image, the region identification information related to the subject appearance image data, and gesture position information representing a position on which the gesture operation has been performed included in the gesture information outputted according to the gesture operation while the subject appearance image is displayed;
an obtaining condition identification means for identifying a medical information obtaining condition for obtaining medical information of the subject corresponding to the gesture operation performed while the subject appearance image is displayed based on the gesture type and gesture region;
a medical information obtaining means for obtaining medical information satisfying the identified medical information obtaining condition from the medical information supply apparatus; and
a second display control means for displaying the obtained medical information on the display means.

15. The medical information display system of Claim 14, wherein the medical information supply apparatus comprises:
a medical information storage means storing a plurality of sets of medical information in a data structure that allows selection of medical information based on a given medical information obtaining condition;
an obtaining condition receiving means for receiving a medical information obtaining condition from the medical information display apparatus;
a medical information retrieval means for obtaining medical information satisfying the received medical information obtaining condition from the medical information storage means; and
a medical information transmission means for transmitting the obtained medical information to the medical information display apparatus that has transmitted the medical information obtaining condition.

16. A medical information display method, comprising:
a step of displaying a subject appearance image representing an appearance of a subject at a predetermined display position of the display means based on image data of the subject appearance image, wherein each position of the image is related to region identification information for identifying a region of the subject;
a step of detecting a gesture operation performed on a display surface of the display means while the subject appearance image is displayed and outputting gesture information representing a content of the detected gesture operation;
a step of determining a gesture type representing to which of a plurality of predetermined gesture operation patterns the detected gesture operation correspond based on the outputted gesture information;
a step of identifying a gesture region which is a region of the subject corresponding to the detected gesture operation based on information of the display position of the subject appearance image, the region identification information related to the subject appearance image data, and gesture position information representing a position on which the gesture operation has been performed included in the gesture information outputted according to the gesture operation;
a step of identifying a medical information obtaining condition for obtaining medical information of the subject corresponding to the gesture operation performed while the subject appearance image is displayed based on the gesture type and gesture region;
a step of selectively obtaining medical information satisfying the identified medical information obtaining condition from a medical information storage means storing a plurality of sets of medical information; and
a step of displaying the obtained medical information on the display means.

17. A medical information display control program for causing a computer to perform:
a step of displaying a subject appearance image representing an appearance of a subject at a predetermined display position of the display means based on image data of the subject appearance image, wherein each position of the image is related to region identification information for identifying a region of the subject;
a step of detecting a gesture operation performed on a display surface of the display means while the subject appearance image is displayed and outputting gesture information representing a content of the detected gesture operation;
a step of determining a gesture type representing to which of a plurality of predetermined gesture operation patterns the detected gesture operation correspond based on the outputted gesture information;
a step of identifying a gesture region which is a region of the subject corresponding to the detected gesture operation based on information of the display position of the subject appearance image, the region identification information related to the subject appearance image data, and gesture position information representing a position on which the gesture operation has been performed included in the gesture information outputted according to the gesture operation;
a step of identifying a medical information obtaining condition for obtaining medical information of the subject corresponding to the gesture operation performed while the subject appearance image is displayed based on the gesture type and gesture region;
a step of selectively obtaining medical information satisfying the identified medical information obtaining condition from a medical information storage means storing a plurality of sets of medical information; and
a step of displaying the obtained medical information on the display means.
